# EUROPEAN PATENT APPLICATION

(11) **EP 2 526 972 A1**
(43) Date of publication of application: **28.11.2012**
(21) Application number: 12181231.7
(22) Date of filing: 18.07.2008
(51) Int. Cl.: A61K 47/48, A61P 31/12, C07K 14/16

(54) **A conjugate of an antibody against CD4 and antifusogenic peptides**

(30) Priority: 20.07.2007 EP 07014335
(62) Divisional of application: 08784874.3
(71) Applicant: F. Hoffmann-La Roche AG, 4070 Basel (CH)
(72) Inventor: Fischer, Stephan, 82362 Weilheim (DE); Kopetzki, Erhard, 82377 Penzberg (DE); Ries, Stefan, 82377 Penzberg (DE); Sankuratri, Suryanarayana, San Jose, California, CA California 95129 (US)
(74) Representative: Burger, Alexander

(57) **Abstract**

The current invention is related to a conjugate comprising two or more antifusogenic peptides and an anti-CD4 antibody (mAb CD4) characterized in that one to eight antifusogenic peptides are each conjugated to one terminus of the heavy and/or light chains of said anti-CD4 antibody and to the pharmaceutical use of said conjugate.

SEQUENCE LISTING

<110>  F. Hoffmann-La Roche AG

<120>  A conjugate of an antibody against CD4 and antifusogenic peptides

<130>  24395 EP2

<150>  EP07014335.9
<151>  2007-07-20

<160>  83

<170>  PatentIn version 3.5

<210>  1
<211>  112
<212>  PRT
<213>  Mus musculus

<400>  1

Asp Ile Val Met Thr Gln Ser Pro Ser Ser Leu Ala Val Ser Val Gly
1               5                   10                  15

Glu Lys Val Thr Met Ile Cys Lys Ser Ser Gln Ser Leu Leu Tyr Ser
            20                  25                  30

Thr Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Thr Gly Ser Gly Ser Gly Thr Asp Phe Thr Leu Thr
65                  70                  75                  80

Ile Ser Ser Val Lys Ala Glu Asp Leu Ala Val Tyr Tyr Cys Gln Gln
                85                  90                  95

Tyr Tyr Ser Tyr Arg Thr Phe Gly Gly Gly Thr Lys Leu Glu Ile Lys
            100                 105                 110

<210>  2
<211>  112
<212>  PRT
<213>  Artificial

<220>
<223>  chimeric light chain variable domain

<400>  2

Asp Ile Val Met Thr Gln Ser Pro Asp Ser Leu Ala Val Ser Leu Gly
1               5                   10                  15

Glu Arg Ala Thr Ile Asn Cys Lys Ser Ser Gly Ser Leu Leu Tyr Ser

                20                  25                  30

Thr Asn Gln Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Tyr Arg Thr Phe Gly Arg Gly Thr Lys Leu Glu Ile Lys
            100                 105                 110

<210>  3
<211>  241
<212>  PRT
<213>  Artificial

<220>
<223>  chimeric light chain

<400>  3

Met Asp Met Arg Val Pro Ala Gln Leu Leu Gly Leu Leu Leu Leu Trp
1               5                   10                  15

Leu Pro Gly Ala Arg Gly Asp Ile Val Met Thr Gln Ser Pro Asp Ser
            20                  25                  30

Leu Ala Val Ser Leu Gly Glu Arg Ala Thr Ile Asn Cys Lys Ser Ser
        35                  40                  45

Gly Ser Leu Leu Tyr Ser Thr Asn Gln Lys Asn Tyr Leu Ala Trp Tyr
    50                  55                  60

Gln Gln Lys Pro Gly Gln Pro Pro Lys Leu Leu Ile Tyr Trp Ala Ser
65                  70                  75                  80

Thr Arg Glu Ser Gly Val Pro Asp Arg Phe Ser Gly Ser Gly Ser Gly
                85                  90                  95

Thr Asp Phe Thr Leu Thr Ile Ser Ser Leu Gln Ala Glu Asp Val Ala
            100                 105                 110

Val Tyr Tyr Cys Gln Gln Tyr Tyr Ser Tyr Arg Thr Phe Gly Arg Gly
        115                 120                 125

Thr Lys Leu Glu Ile Lys Arg Thr Val Ala Ala Pro Ser Val Phe Ile
    130                 135                 140

Phe Pro Pro Ser Asp Glu Gln Leu Lys Ser Gly Thr Ala Ser Val Val
145                 150                 155                 160

Cys Leu Leu Asn Asn Phe Tyr Pro Arg Glu Ala Lys Val Gln Trp Lys
                165                 170                 175

Val Asp Asn Ala Leu Gln Ser Gly Asn Ser Gln Glu Ser Val Thr Glu
            180                 185                 190

Gln Asp Ser Lys Asp Ser Thr Tyr Ser Leu Ser Ser Thr Leu Thr Leu
        195                 200                 205

Ser Lys Ala Asp Tyr Glu Lys His Lys Val Tyr Ala Cys Glu Val Thr
    210                 215                 220

His Gln Gly Leu Ser Ser Pro Val Thr Lys Ser Phe Asn Arg Gly Glu
225                 230                 235                 240

Cys

<210> 4
<211> 112
<212> PRT
<213> Artificial

<220>
<223> chimeric light chain variable domain

<400> 4

Asp Ile Val Met Thr Gln Ser Pro Asp Ser Leu Ala Val Ser Leu Gly
1               5                   10                  15

Glu Arg Val Thr Met Asn Cys Lys Ser Ser Gln Ser Leu Leu Tyr Ser
            20                  25                  30

Thr Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Val Gln Ala Glu Asp Val Ala Val Tyr Tyr Cys Gln Gln
                85                  90                  95

Tyr Tyr Ser Tyr Arg Thr Phe Gly Gly Gly Thr Lys Leu Glu Ile Lys
            100                 105                 110

<210>  5
<211>  108
<212>  PRT
<213>  Artificial

<220>
<223>  chimeric light chain variable domain

<400>  5

Asp Ile Gln Met Thr Gln Ser Pro Ser Ser Leu Ser Ala Ser Val Gly
1               5                   10                  15

Asp Arg Val Thr Ile Thr Cys Lys Ala Ser Gln Asp Ile Asn Asn Tyr
            20                  25                  30

Ile Ala Trp Tyr Gln His Thr Pro Gly Lys Ala Pro Lys Leu Leu Ile
        35                  40                  45

His Tyr Thr Ser Thr Leu Gln Pro Gly Val Pro Ser Arg Phe Ser Gly
    50                  55                  60

Ser Gly Ser Gly Thr Asp Tyr Thr Phe Thr Ile Ser Ser Leu Gln Pro
65                  70                  75                  80

Glu Asp Ile Ala Thr Tyr Cys Leu Gln Gln Tyr Asp Asn Leu Leu Phe
                85                  90                  95

Thr Phe Gly Gln Gly Thr Lys Leu Gln Ile Thr Arg
            100                 105

<210>  6
<211>  112
<212>  PRT
<213>  Artificial

<220>
<223>  chimeric light chain variable domain

<400>  6

Asp Ile Val Met Thr Gln Ser Pro Asp Ser Leu Ala Val Ser Leu Gly
1               5                   10                  15

Glu Arg Val Thr Met Asn Cys Lys Ser Ser Gln Ser Leu Leu Tyr Ser
            20                  25                  30

Thr Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Val Gln Ala Glu Asp Val Ala Val Tyr Tyr Cys Gln Gln
                85                  90                  95

Tyr Tyr Ser Tyr Arg Thr Phe Gly Gly Gly Thr Lys Leu Glu Ile Lys
            100                 105                 110

<210>  7
<211>  122
<212>  PRT
<213>  Mus musculus

<400>  7

Glu Val Lys Leu Gln Glu Ser Gly Pro Glu Leu Val Lys Pro Gly Ala
1               5                   10                  15

Ser Val Lys Met Ser Cys Lys Ala Ser Gly Tyr Thr Phe Thr Ser Tyr
            20                  25                  30

Val Ile His Trp Val Arg Gln Lys Pro Gly Gln Gly Leu Asp Trp Ile
        35                  40                  45

Gly Tyr Ile Asn Pro Tyr Asn Asp Gly Thr Asp Tyr Asp Glu Lys Phe
    50                  55                  60

Lys Gly Lys Ala Thr Leu Thr Ser Asp Lys Ser Ser Ser Thr Ala Tyr
65                  70                  75                  80

Met Glu Leu Ser Ser Leu Thr Ser Glu Asp Ser Ala Val Tyr Tyr Cys
                85                  90                  95

Ala Arg Glu Lys Asp Asn Tyr Ala Thr Gly Ala Trp Phe Ala Tyr Trp
            100                 105                 110

Gly Gln Gly Thr Thr Val Thr Val Ser Ser
        115                 120

<210>  8
<211>  122
<212>  PRT
<213>  Artificial

<220>
<223>  humanized heavy chain variable domain

<400>  8

Gln Val Gln Leu Gln Glu Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Val Ile His Trp Val Arg Gln Ala Pro Gly Gln Gly Leu Glu Trp Ile
        35                  40                  45

Gly Tyr Ile Asn Pro Tyr Asn Asp Gly Thr Asp Tyr Asp Glu Lys Phe
    50                  55                  60

Lys Gly Lys Ala Thr Val Thr Leu Asp Pro Ser Thr Asn Thr Ala Tyr
65                  70                  75                  80

Met Glu Leu Ser Ser Leu Arg Ser Glu Asp Thr Ala Val Tyr Tyr Cys
                85                  90                  95

Ala Arg Glu Lys Asp Asn Tyr Ala Thr Gly Ala Trp Phe Ala Tyr Trp
            100                 105                 110

Gly Gln Gly Thr Leu Val Thr Val Ser Ser
        115                 120

<210>  9
<211>  467
<212>  PRT
<213>  Artificial

<220>
<223>  chimeric heavy chain

<400>  9

Met Asp Trp Thr Trp Arg Val Phe Cys Leu Leu Ala Val Ala Pro Gly
1               5                   10                  15

Ala His Ser Gln Val Gln Leu Gln Glu Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Ser Tyr Val Ile His Trp Val Arg Gln Ala Pro Gly Gln Gly Leu
    50                  55                  60

Glu Trp Ile Gly Tyr Ile Asn Pro Tyr Asn Asp Gly Thr Asp Tyr Asp
65                  70                  75                  80

Glu Lys Phe Lys Gly Lys Ala Thr Val Thr Leu Asp Pro Ser Thr Asn
                85                  90                  95

Thr Ala Tyr Met Glu Leu Ser Ser Leu Arg Ser Glu Asp Thr Ala Val
            100                 105                 110

Tyr Tyr Cys Ala Arg Glu Lys Asp Asn Tyr Ala Thr Gly Ala Trp Phe
        115                 120                 125

Ala Tyr Trp Gly Gln Gly Thr Leu Val Thr Val Ser Ser Ala Ser Thr
    130                 135                 140

Lys Gly Pro Ser Val Phe Pro Leu Ala Pro Cys Ser Arg Ser Thr Ser
145                 150                 155                 160

Glu Ser Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Lys Thr Tyr Thr Cys
    210                 215                 220

Asn Val Asp His Lys Pro Ser Asn Thr Lys Val Asp Lys Arg Val Glu
225                 230                 235                 240

Ser Lys Tyr Gly Pro Pro Cys Pro Ser Cys Pro Ala Pro Glu Phe Leu
                245                 250                 255

Gly Gly Pro Ser Val Phe Leu Phe Pro Pro Lys Pro Lys Asp Thr Leu
            260                 265                 270

Met Ile Ser Arg Thr Pro Glu Val Thr Cys Val Val Val Asp Val Ser
        275                 280                 285

Gln Glu Asp Pro Glu Val Gln Phe Asn Trp Tyr Val Asp Gly Val Glu
    290                 295                 300

Val His Asn Ala Lys Thr Lys Pro Arg Glu Glu Gln Phe Asn Ser Thr
305                 310                 315                 320

Tyr Arg Val Val Ser Val Leu Thr Val Leu His Gln Asp Trp Leu Asn
                325                 330                 335

Gly Lys Glu Tyr Lys Cys Lys Val Ser Asn Lys Gly Leu Pro Ser Ser
            340                 345                 350

Ile Glu Lys Thr Ile Ser Lys Ala Lys Gln Pro Arg Glu Pro Gln Val
        355                 360                 365

Tyr Thr Leu Pro Pro Ser Gln Glu Glu Met Thr Lys Asn Gln Val Ser
    370                 375                 380

Leu Thr Cys Leu Val Lys Gly Phe Tyr Pro Ser Asp Ile Ala Val Glu
385                 390                 395                 400

Trp Glu Ser Asn Gly Gln Pro Glu Asn Asn Tyr Lys Thr Thr Pro Pro
                405                 410                 415

Val Leu Asp Ser Asp Gly Ser Phe Phe Leu Tyr Ser Arg Leu Thr Val
            420                 425                 430

Asp Lys Ser Arg Trp Gln Glu Gly Asn Val Phe Ser Cys Ser Val Met
        435                 440                 445

His Glu Ala Leu His Asn His Tyr Thr Gln Lys Ser Leu Ser Leu Ser
    450                 455                 460

Leu Gly Lys
465

<210>  10
<211>  122
<212>  PRT
<213>  Artificial

<220>
<223>  chimeric heavy chain variable domain

<400>  10

Gln Val Gln Leu Gln Gln Ser Gly Pro Glu Val Val Lys Pro Gly Ala
1               5                   10                  15

Ser Val Lys Met Ser Cys Lys Ala Ser Gly Tyr Thr Phe Thr Ser Tyr
            20                  25                  30

Val Ile His Trp Val Arg Gln Lys Pro Gly Gln Gly Leu Asp Trp Ile
        35                  40                  45

Gly Tyr Ile Asn Pro Tyr Asn Asp Gly Thr Asp Tyr Asp Glu Lys Phe
    50                  55                  60

Lys Gly Lys Ala Thr Leu Thr Ser Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Glu Lys Asp Asn Tyr Ala Thr Gly Ala Trp Phe Ala Tyr Trp
            100                 105                 110

Gly Gln Gly Thr Leu Val Thr Val Ser Ser
        115                 120

<210>  11
<211>  120
<212>  PRT
<213>  Artificial

<220>
<223>  chimeric heavy chain variable domain

<400>  11

Gln Val Gln Leu Val Glu Ser Gly Gly Gly Val Val Gln Pro Gly Arg
1               5                   10                  15

Ser Leu Arg Leu Ser Cys Ser Ala Ser Gly Phe Thr Phe Ser Asn Tyr
            20                  25                  30

Ala Met Ser Trp Val Arg Gln Ala Pro Gly Lys Gly Leu Glu Trp Val
        35                  40                  45

Ala Ala Ile Ser Asp His Ser Thr Asn Thr Tyr Tyr Pro Asp Ser Val
    50                  55                  60

Lys Gly Arg Phe Thr Ile Ser Arg Asp Asn Ser Lys Asn Thr Leu Phe
65                  70                  75                  80

Leu Gln Met Asp Ser Leu Arg Pro Glu Asp Thr Ala Val Tyr Tyr Cys
                85                  90                  95

Ala Arg Lys Tyr Gly Gly Asp Tyr Asp Pro Phe Asp Tyr Trp Gly Gln
            100                 105                 110

Gly Thr Pro Val Thr Val Ser Ser
        115                 120

<210>  12
<211>  122
<212>  PRT
<213>  Artificial

<220>
<223>  chimeric heavy chain variable domain

<400>  12

Gln Val Gln Leu Gln Gln Ser Gly Pro Glu Val Val Lys Pro Gly Ala
1               5                   10                  15

Ser Val Lys Met Ser Cys Lys Ala Ser Gly Tyr Thr Phe Thr Ser Tyr
            20                  25                  30

Val Ile His Trp Val Arg Gln Lys Pro Gly Gln Gly Leu Asp Trp Ile
        35                  40                  45

Gly Tyr Ile Asn Pro Tyr Asn Asp Gly Thr Asp Tyr Asp Glu Lys Phe
    50                  55                  60

Lys Gly Lys Ala Thr Leu Thr Ser Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Glu Lys Asp Asn Tyr Ala Thr Gly Ala Trp Phe Ala Tyr Trp
            100                 105                 110

Gly Gln Gly Thr Leu Val Thr Val Ser Ser
        115                 120

<210>  13
<211>  17
<212>  PRT
<213>  Mus musculus

<400>  13

Lys Ser Ser Gln Ser Leu Leu Tyr Ser Thr Asn Gln Lys Asn Tyr Leu
1               5                   10                  15

Ala

<210>  14
<211>  17
<212>  PRT
<213>  Artificial

<220>
<223>  CDR

<400>  14

Lys Ser Ser Gly Ser Leu Leu Tyr Ser Thr Asn Gln Lys Asn Tyr Leu
1               5                   10                  15

Ala

<210>  15
<211>  17
<212>  PRT
<213>  Artificial

<220>
<223>  CDR

<400>  15

Lys Ser Ser Gln Ser Leu Leu Tyr Ser Thr Asn Gln Lys Asn Tyr Leu
1               5                   10                  15

Ala

<210>  16
<211>  9
<212>  PRT
<213>  Artificial

<220>
<223>  CDR

<400>  16

Lys Ala Ser Gln Asp Ile Asn Asn Tyr
1               5

<210>  17
<211>  7
<212>  PRT
<213>  Mus musculus

<400>  17

Trp Ala Ser Thr Arg Glu Ser
1               5

<210>  18
<211>  7
<212>  PRT
<213>  Mus musculus

<400>  18

Trp Ala Ser Thr Arg Glu Ser
1               5

<210>  19
<211>  11
<212>  PRT
<213>  Mus musculus

<400>  19

Tyr Thr Ser Thr Leu Gln Pro Gly Val Pro Ser
1               5                   10

<210>  20
<211>  8
<212>  PRT
<213>  Mus musculus

<400>  20

Gln Gln Tyr Tyr Ser Tyr Arg Thr
1               5


<210>  21
<211>  8
<212>  PRT
<213>  Mus musculus

<400>  21

Gln Gln Tyr Tyr Ser Tyr Arg Thr
1               5


<210>  22
<211>  6
<212>  PRT
<213>  Mus musculus

<400>  22

Tyr Asp Asn Leu Leu Phe
1               5


<210>  23
<211>  5
<212>  PRT
<213>  Mus musculus

<400>  23

Ser Tyr Val Ile His
1               5


<210>  24
<211>  5
<212>  PRT
<213>  Mus musculus

<400>  24

Ser Tyr Val Ile His
1               5


<210>  25
<211>  10
<212>  PRT
<213>  Mus musculus

<400>  25

Gly Phe Thr Phe Ser Asn Tyr Ala Met Ser
1               5                   10


<210>  26
<211>  17
<212>  PRT

<213>  Mus musculus

<400>  26

Tyr Ile Asn Pro Tyr Asn Asp Gly Thr Asp Tyr Asp Glu Lys Phe Lys
1               5                   10                  15

Gly

<210>  27
<211>  17
<212>  PRT
<213>  Mus musculus

<400>  27

Tyr Ile Asn Pro Tyr Asn Asp Gly Thr Asp Tyr Asp Glu Lys Phe Lys
1               5                   10                  15

Gly

<210>  28
<211>  12
<212>  PRT
<213>  Mus musculus

<400>  28

Ala Ile Ser Asp His Ser Thr Asn Thr Tyr Tyr Pro
1               5                   10

<210>  29
<211>  13
<212>  PRT
<213>  Mus musculus

<400>  29

Glu Lys Asp Asn Tyr Ala Thr Gly Ala Trp Phe Ala Tyr
1               5                   10

<210>  30
<211>  13
<212>  PRT
<213>  Mus musculus

<400>  30

Glu Lys Asp Asn Tyr Ala Thr Gly Ala Trp Phe Ala Tyr
1               5                   10

<210>  31
<211>  11
<212>  PRT
<213>  Mus musculus

<400>  31

Ala Arg Lys Tyr Gly Gly Asp Tyr Asp Pro Phe
1               5                   10


<210>  32
<211>  330
<212>  PRT
<213>  Homo sapiens

<400>  32

<210>  33
<211>  327
<212>  PRT
<213>  Homo sapiens

<400>  33

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  34
<211>  107
<212>  PRT
<213>  Homo sapiens

<400>  34

<210>  35
<211>  34
<212>  PRT
<213>  Artificial

<220>
<223>  C34

<400>  35

Trp Met Glu Trp Asp Arg Glu Ile Asn Asn Tyr Thr Ser Leu Ile His
1               5                   10                  15

Ser Leu Ile Glu Glu Ser Gln Asn Gln Gln Glu Lys Asn Glu Gln Glu
            20                  25                  30

Leu Leu

<210>  36
<211>  36
<212>  PRT
<213>  Artificial

<220>
<223>  T20

<400>  36

Tyr Thr Ser Leu Ile His Ser Leu Ile Glu Glu Ser Gln Asn Gln Gln
1               5                   10                  15

Glu Lys Asn Glu Gln Glu Leu Leu Glu Leu Asp Lys Trp Ala Ser Leu

20                  25                  30

Trp Asn Trp Phe
        35

<210>  37
<211>  39
<212>  PRT
<213>  Artificial

<220>
<223>  T1249

<400>  37

Trp Gln Glu Trp Glu Gln Lys Ile Thr Ala Leu Leu Glu Gln Ala Gln
1               5                   10                  15

Ile Gln Gln Glu Lys Asn Glu Tyr Glu Leu Gln Lys Leu Asp Lys Trp
            20                  25                  30

Ala Ser Leu Trp Glu Trp Phe
        35

<210>  38
<211>  36
<212>  PRT
<213>  Artificial

<220>
<223>  T651

<400>  38

Met Thr Trp Met Glu Trp Asp Arg Glu Ile Asn Asn Tyr Thr Ser Leu
1               5                   10                  15

Ile His Ser Leu Ile Glu Glu Ser Gln Asn Gln Gln Glu Lys Asn Glu
            20                  25                  30

Gln Glu Leu Leu
        35

<210>  39
<211>  38
<212>  PRT
<213>  Artificial

<220>
<223>  T2635

<400>  39

Thr Thr Trp Glu Ala Trp Asp Arg Ala Ile Ala Glu Tyr Ala Ala Arg
1               5                   10                  15

Ile Glu Ala Leu Ile Arg Ala Ala Gln Glu Gln Gln Glu Lys Asn Glu
            20                  25                  30

Ala Ala Leu Arg Glu Leu
        35

<210> 40
<211> 36
<212> PRT
<213> Artificial

<220>
<223> N36

<400> 40

Ser Gly Ile Val Gln Gln Gln Asn Asn Leu Leu Arg Ala Ile Glu Ala
1               5                   10                  15

Gln Gln His Leu Leu Gln Leu Thr Val Trp Gly Ile Lys Gln Leu Gln
            20                  25                  30

Ala Arg Ile Leu
        35

<210> 41
<211> 38
<212> PRT
<213> Artificial

<220>
<223> DP107

<400> 41

Asn Asn Leu Leu Arg Ala Ile Glu Ala Gln Gln His Leu Leu Gln Leu
1               5                   10                  15

Thr Val Trp Gly Ile Lys Gln Leu Gln Ala Arg Ile Leu Ala Val Glu
            20                  25                  30

Arg Tyr Leu Lys Asp Gln
        35

<210> 42
<211> 37
<212> PRT
<213> Artificial

<220>
<223> afp-1

<400> 42

Asn Met Thr Trp Met Glu Trp Asp Arg Glu Ile Asn Gln Tyr Thr Ser
1               5                   10                  15

Leu Ile His Ser Leu Ile Glu Glu Ser Gln Asn Gln Gln Glu Lys Asn
            20                  25                  30

Glu Gln Glu Leu Leu
        35

<210>  43
<211>  123
<212>  PRT
<213>  Artificial

<220>
<223>  HIV-1 gp41 ectodomain variant single mutant: I568P

<400>  43

Val Gln Ala Arg Gln Leu Leu Ser Gly Ile Val Gln Gln Gln Asn Asn
1               5                   10                  15

Leu Leu Arg Ala Ile Glu Gly Gln Gln His Leu Leu Gln Leu Thr Val
            20                  25                  30

Trp Gly Pro Lys Gln Leu Gln Ala Arg Ile Leu Ala Val Glu Arg Tyr
        35                  40                  45

Leu Lys Asp Gln Gln Leu Leu Gly Ile Trp Gly Cys Ser Gly Lys Leu
    50                  55                  60

Ile Cys Thr Thr Ala Val Pro Trp Asn Ala Ser Trp Ser Asn Lys Ser
65                  70                  75                  80

Leu Glu Gln Ile Trp Asn Asn Met Thr Trp Met Glu Trp Asp Arg Glu
                85                  90                  95

Ile Asn Asn Tyr Thr Ser Leu Ile His Ser Leu Ile Glu Glu Ser Gln
            100                 105                 110

Asn Gln Gln Glu Lys Asn Glu Gln Glu Leu Leu
        115                 120

<210>  44
<211>  132
<212>  PRT
<213>  Artificial

<220>
<223>  HIV-1 gp41 ectodomain variant quadruple mutant: I568P, L550E,
       L566E, I580E

<400>  44

Met Gly Ala Ala Ser Met Thr Leu Thr Val Gln Ala Arg Gln Leu Leu
1               5                   10                  15

Ser Gly Ile Val Gln Gln Gln Asn Asn Glu Leu Arg Ala Ile Glu Gly
            20                  25                  30

Gln Gln His Leu Glu Gln Leu Thr Val Trp Gly Pro Lys Gln Leu Gln
        35                  40                  45

Ala Arg Glu Leu Ala Val Glu Arg Tyr Leu Lys Asp Gln Gln Leu Leu
    50                  55                  60

Gly Ile Trp Gly Cys Ser Gly Lys Leu Ile Cys Thr Thr Ala Val Pro
65                  70                  75                  80

Trp Asn Ala Ser Trp Ser Asn Lys Ser Leu Glu Gln Ile Trp Asn Asn
                85                  90                  95

Met Thr Trp Met Glu Trp Asp Arg Glu Ile Asn Asn Tyr Thr Ser Leu
            100                 105                 110

Ile His Ser Leu Ile Glu Glu Ser Gln Asn Gln Gln Glu Lys Asn Glu
        115                 120                 125

Gln Glu Leu Leu
    130

<210>  45
<211>  345
<212>  PRT
<213>  Human immunodeficiency virus

<400>  45

Ala Val Gly Ile Gly Ala Leu Phe Leu Gly Phe Leu Gly Ala Ala Gly
1               5                   10                  15

Ser Thr Met Gly Ala Ala Ser Met Thr Leu Thr Val Gln Ala Arg Gln
            20                  25                  30

Leu Leu Ser Gly Ile Val Gln Gln Gln Asn Asn Leu Leu Arg Ala Ile
        35                  40                  45

Glu Ala Gln Gln His Leu Leu Gln Leu Thr Val Trp Gly Ile Lys Gln
    50                  55                  60

Leu Gln Ala Arg Ile Leu Ala Val Glu Arg Tyr Leu Lys Asp Gln Gln
65                  70                  75                  80

Leu Leu Gly Ile Trp Gly Cys Ser Gly Lys Leu Ile Cys Thr Thr Ala
                85                  90                  95

Val Pro Trp Asn Ala Ser Trp Ser Asn Lys Ser Leu Glu Gln Ile Trp

            100                 105                 110

Asn His Thr Thr Trp Met Glu Trp Asp Arg Glu Ile Asn Asn Tyr Thr
        115                 120                 125

Ser Leu Ile His Ser Leu Ile Glu Glu Ser Gln Asn Gln Gln Glu Lys
    130                 135                 140

Asn Glu Gln Glu Leu Leu Glu Leu Asp Lys Trp Ala Ser Leu Trp Asn
145                 150                 155                 160

Trp Phe Asn Ile Thr Asn Trp Leu Trp Tyr Ile Lys Leu Phe Ile Met
                165                 170                 175

Ile Val Gly Gly Leu Val Gly Leu Arg Ile Val Phe Ala Val Leu Ser
            180                 185                 190

Ile Val Asn Arg Val Arg Gln Gly Tyr Ser Pro Leu Ser Phe Gln Thr
        195                 200                 205

His Leu Pro Thr Pro Arg Gly Pro Asp Arg Pro Glu Gly Ile Glu Glu
    210                 215                 220

Glu Gly Gly Glu Arg Asp Arg Asp Arg Ser Ile Arg Leu Val Asn Gly
225                 230                 235                 240

Ser Leu Ala Leu Ile Trp Asp Asp Leu Arg Ser Leu Cys Leu Phe Ser
                245                 250                 255

Tyr His Arg Leu Arg Asp Leu Leu Leu Ile Val Thr Arg Ile Val Glu
            260                 265                 270

Leu Leu Gly Arg Arg Gly Trp Glu Ala Leu Lys Tyr Trp Trp Asn Leu
        275                 280                 285

Leu Gln Tyr Trp Ser Gln Glu Leu Lys Asn Ser Ala Val Ser Leu Leu
    290                 295                 300

Asn Ala Thr Ala Ile Ala Val Ala Glu Gly Thr Asp Arg Val Ile Glu
305                 310                 315                 320

Val Val Gln Gly Ala Cys Arg Ala Ile Arg His Ile Pro Arg Arg Ile
                325                 330                 335

Arg Gln Gly Leu Glu Arg Ile Leu Leu
            340                 345

<210> 46
<211> 7

<212>  PRT
<213>  Artificial

<220>
<223>  linker 1

<400>  46

Leu Ser Leu Ser Pro Gly Lys
1               5


<210>  47
<211>  8
<212>  PRT
<213>  Artificial

<220>
<223>  linker 2

<400>  47

Leu Ser Pro Asn Arg Gly Glu Cys
1               5


<210>  48
<211>  15
<212>  PRT
<213>  Artificial

<220>
<223>  linker 3

<400>  48

Gly Gln Gln Gln Gln Gly Gln Gln Gln Gln Gly Gln Gln Gln Gln
1               5                   10                  15


<210>  49
<211>  16
<212>  PRT
<213>  Artificial

<220>
<223>  linker 4

<400>  49

Gly Gln Gln Gln Gln Gly Gln Gln Gln Gln Gly Gln Gln Gln Gln Gly
1               5                   10                  15


<210>  50
<211>  18
<212>  PRT
<213>  Artificial

<220>
<223>  linker 5

<400>  50

Gly Gln Gln Gln Gln Gly Gln Gln Gln Gln Gly Gln Gln Gln Gln Gly

1               5                   10                  15

Asn Asn

<210>  51
<211>  16
<212>  PRT
<213>  Artificial

<220>
<223>  linker 6

<400>  51

Gly Gly Gly Ser Gly Gly Gly Gly Ser Gly Gly Gly Gly Ser Gly Gly
1               5                   10                  15

<210>  52
<211>  16
<212>  PRT
<213>  Artificial

<220>
<223>  linker 7

<400>  52

Gly Gly Gly Ser Gly Gly Gly Gly Ser Gly Gly Gly Gly Ser Gly Asn
1               5                   10                  15

<210>  53
<211>  15
<212>  PRT
<213>  Artificial

<220>
<223>  linker 8

<400>  53

Ser Gly Gly Gly Gly Ser Gly Gly Gly Gly Ser Gly Gly Gly Gly
1               5                   10                  15

<210>  54
<211>  16
<212>  PRT
<213>  Artificial

<220>
<223>  linker 9

<400>  54

Ser Gly Gly Gly Gly Ser Gly Gly Gly Gly Ser Gly Gly Gly Gly Gly
1               5                   10                  15

<210>  55
<211>  17

<212>  PRT
<213>  Artificial

<220>
<223>  linker 10

<400>  55

Gly Ser Gly Gly Gly Gly Ser Gly Gly Gly Gly Ser Gly Gly Gly Gly
1               5                   10                  15

Thr

<210>  56
<211>  17
<212>  PRT
<213>  Artificial

<220>
<223>  linker 11

<400>  56

Ser Gly Gly Gly Gly Ser Gly Gly Gly Gly Ser Gly Gly Gly Gly Gly
1               5                   10                  15

Gly

<210>  57
<211>  18
<212>  PRT
<213>  Artificial

<220>
<223>  linker 12

<400>  57

Ser Gly Gly Gly Gly Ser Gly Gly Gly Gly Ser Gly Gly Gly Gly Gly
1               5                   10                  15

Gly Thr

<210>  58
<211>  18
<212>  PRT
<213>  Artificial

<220>
<223>  linker 13

<400>  58

Ser Gly Gly Gly Gly Ser Gly Gly Gly Gly Ser Gly Gly Gly Gly Gly
1               5                   10                  15

Gly Asn

<210> 59
<211> 18
<212> PRT
<213> Artificial

<220>
<223> linker 14

<400> 59

Ser Gly Gly Gly Gly Ser Gly Gly Gly Gly Ser Gly Gly Gly Gly Gly
1               5                   10                  15

Ala Ser

<210> 60
<211> 25
<212> PRT
<213> Artificial

<220>
<223> linker 15

<400> 60

Ser Gly Gly Gly Gly Ser Gly Gly Gly Gly Ser Gly Gly Gly Gly Ser
1               5                   10                  15

Gly Gly Gly Gly Ser Gly Gly Gly Gly
            20                  25

<210> 61
<211> 26
<212> PRT
<213> Artificial

<220>
<223> linker 16

<400> 61

Ser Gly Gly Gly Gly Ser Gly Gly Gly Gly Ser Gly Gly Gly Gly Ser
1               5                   10                  15

Gly Gly Gly Gly Ser Gly Gly Gly Gly Gly
            20                  25

<210> 62
<211> 27
<212> PRT
<213> Artificial

<220>

<223>  linker 17

<400>  62

Ser Gly Gly Gly Gly Ser Gly Gly Gly Gly Ser Gly Gly Gly Gly Ser
1               5                   10                  15

Gly Gly Gly Gly Ser Gly Gly Gly Gly Gly Gly
            20                  25

<210>  63
<211>  28
<212>  PRT
<213>  Artificial

<220>
<223>  linker 18

<400>  63

Ser Gly Gly Gly Gly Ser Gly Gly Gly Gly Ser Gly Gly Gly Gly Ser
1               5                   10                  15

Gly Gly Gly Gly Ser Gly Gly Gly Gly Gly Ala Ser
            20                  25

<210>  64
<211>  17
<212>  PRT
<213>  Artificial

<220>
<223>  linker 19

<400>  64

Gly Ser Ser Ser Ser Ser Ser Ser Ser Ser Ser Ser Ser Ser Ser Ser
1               5                   10                  15

Gly

<210>  65
<211>  19
<212>  PRT
<213>  Artificial

<220>
<223>  linker 20

<400>  65

Gly Ser Ser Ser Ser Ser Ser Ser Ser Ser Ser Ser Ser Ser Ser Ser
1               5                   10                  15

Gly Ala Ser

<210>  66
<211>  18
<212>  PRT
<213>  Artificial

<220>
<223>  linker 24

<400>  66

Gly Gly Gly Gly Ser Gly Gly Gly Gly Ser Gly Gly Gly Gly Ser Gly
1               5                   10                  15

Ala Ser

<210>  67
<211>  16
<212>  PRT
<213>  Artificial

<220>
<223>  linker 25

<400>  67

Gly Gly Gly Gly Ser Gly Gly Gly Gly Ser Gly Gly Gly Gly Ser Gly
1               5                   10                  15

<210>  68
<211>  26
<212>  PRT
<213>  Artificial

<220>
<223>  linker 26

<400>  68

Gly Gly Gly Gly Ser Gly Gly Gly Gly Ser Gly Gly Gly Gly Ser Gly
1               5                   10                  15

Gly Gly Gly Ser Gly Gly Gly Gly Ser Gly
            20                  25

<210>  69
<211>  17
<212>  PRT
<213>  Artificial

<220>
<223>  linker 27

<400>  69

Gly Gly Gly Gly Ser Gly Gly Gly Gly Ser Gly Gly Gly Gly Ser Gly
1               5                   10                  15

Gly

<210> 70
<211> 27
<212> PRT
<213> Artificial

<220>
<223> linker 28

<400> 70

Gly Gly Gly Gly Ser Gly Gly Gly Gly Ser Gly Gly Gly Gly Ser Gly
1               5                   10                  15

Gly Gly Gly Ser Gly Gly Gly Gly Ser Gly Gly
            20                  25

<210> 71
<211> 6
<212> PRT
<213> Artificial

<220>
<223> linker 29

<400> 71

Leu Ser Leu Ser Gly Gly
1               5

<210> 72
<211> 7
<212> PRT
<213> Artificial

<220>
<223> linker 30

<400> 72

Leu Ser Leu Ser Pro Gly Gly
1               5

<210> 73
<211> 20
<212> PRT
<213> Artificial

<220>
<223> linker 31

<400> 73

Gly Gly Gly Ser Gly Gly Gly Ser Gly Gly Gly Ser Gly Gly Gly Ser
1               5                   10                  15

Gly Gly Gly Ser

            20

<210>  74
<211>  23
<212>  PRT
<213>  Artificial

<220>
<223>  linker 32

<400>  74

Gly Gly Gly Ser Gly Gly Gly Ser Gly Gly Gly Ser Gly Gly Gly Ser
1               5                   10                  15

Gly Gly Gly Ser Gly Gly Gly
            20

<210>  75
<211>  15
<212>  PRT
<213>  Artificial

<220>
<223>  linker 33

<400>  75

Gly Gly Gly Ser Gly Gly Gly Gly Ser Gly Gly Gly Gly Ser Gly
1               5                   10                  15

<210>  76
<211>  16
<212>  PRT
<213>  Artificial

<220>
<223>  linker 34

<400>  76

Gly Gly Gly Ser Gly Gly Gly Gly Ser Gly Gly Gly Gly Ser Gly Gly
1               5                   10                  15

<210>  77
<211>  219
<212>  PRT
<213>  Artificial

<220>
<223>  anti-CD4 antibody light chain

<400>  77

Asp Ile Val Met Thr Gln Ser Pro Asp Ser Leu Ala Val Ser Leu Gly
1               5                   10                  15

Glu Arg Val Thr Met Asn Cys Lys Ser Ser Gln Ser Leu Leu Tyr Ser
            20                  25                  30

Thr Asn Gln Lys Asn Tyr Leu Ala Trp Tyr Gln Gln Lys Pro Gly Gln
        35                  40                  45

Ser Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Val Gln Ala Glu Asp Val Ala Val Tyr Tyr Cys Gln Gln
                85                  90                  95

Tyr Tyr Ser Tyr Arg Thr Phe Gly Gly Gly Thr Lys Leu Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  78
<211>  452
<212>  PRT
<213>  Artificial

<220>
<223>  anti-CD4 antibody heavy chain

<400>  78

Gln Val Gln Leu Gln Gln Ser Gly Pro Glu Val Val Lys Pro Gly Ala
1               5                   10                  15

Ser Val Lys Met Ser Cys Lys Ala Ser Gly Tyr Thr Phe Thr Ser Tyr

20                  25                  30

Val Ile His Trp Val Arg Gln Lys Pro Gly Gln Gly Leu Asp Trp Ile
        35                  40                  45

Gly Tyr Ile Asn Pro Tyr Asn Asp Gly Thr Asp Tyr Asp Glu Lys Phe
    50                  55                  60

Lys Gly Lys Ala Thr Leu Thr Ser Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Glu Lys Asp Asn Tyr Ala Thr Gly Ala Trp Phe Ala Tyr Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Ala Ala
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys
    450

<210>  79
<211>  503
<212>  PRT
<213>  Artificial

<220>
<223>  anti-CD4 antibody heavy chain - afp-1 antifusogenic peptide
       conjugate

<400>  79

Gln Val Gln Leu Gln Gln Ser Gly Pro Glu Val Val Lys Pro Gly Ala
1               5                   10                  15

Ser Val Lys Met Ser Cys Lys Ala Ser Gly Tyr Thr Phe Thr Ser Tyr
            20                  25                  30

Val Ile His Trp Val Arg Gln Lys Pro Gly Gln Gly Leu Asp Trp Ile
        35                  40                  45

Gly Tyr Ile Asn Pro Tyr Asn Asp Gly Thr Asp Tyr Asp Glu Lys Phe
    50                  55                  60

Lys Gly Lys Ala Thr Leu Thr Ser Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Glu Lys Asp Asn Tyr Ala Thr Gly Ala Trp Phe Ala Tyr Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Ala Ala
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr

290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Gly Gly Gly Ser Gly Gly Gly Gly Ser Gly Gly Gly Gly
    450                 455                 460

Ser Gly Asn Met Thr Trp Met Glu Trp Asp Arg Glu Ile Asn Gln Tyr
465                 470                 475                 480

Thr Ser Leu Ile His Ser Leu Ile Glu Glu Ser Gln Asn Gln Gln Glu
                485                 490                 495

Lys Asn Glu Gln Glu Leu Leu
            500

<210>  80
<211>  18
<212>  PRT
<213>  Artificial

<220>
<223>  linker 35

<400>  80

Gly Gly Gly Gly Ser Gly Gly Gly Gly Ser Gly Gly Gly Gly Ser Gly
1               5                   10                  15

Gly Asn

<210>  81
<211>  19
<212>  PRT
<213>  Artificial

<220>
<223>  linker 36

<400>  81

Lys Gly Gly Gly Gly Ser Gly Gly Gly Gly Ser Gly Gly Gly Gly Ser
1               5                   10                  15

Gly Gly Asn

<210>  82
<211>  506
<212>  PRT
<213>  Artificial

<220>
<223>  anti-CD4 antibody heavy chain

<400>  82

Gln Val Gln Leu Gln Gln Ser Gly Pro Glu Val Val Lys Pro Gly Ala
1               5                   10                  15

Ser Val Lys Met Ser Cys Lys Ala Ser Gly Tyr Thr Phe Thr Ser Tyr
            20                  25                  30

Val Ile His Trp Val Arg Gln Lys Pro Gly Gln Gly Leu Asp Trp Ile
        35                  40                  45

Gly Tyr Ile Asn Pro Tyr Asn Asp Gly Thr Asp Tyr Asp Glu Lys Phe
    50                  55                  60

Lys Gly Lys Ala Thr Leu Thr Ser Asp Thr Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Glu Lys Asp Asn Tyr Ala Thr Gly Ala Trp Phe Ala Tyr Trp
            100                 105                 110

Gly Gln Gly Thr Leu Val Thr Val Ser Ser Ala Ser Thr Lys Gly Pro

115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Ala Ala
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys Gly Gly Gly Gly Ser Gly Gly Gly Gly Ser Gly Gly
    450                 455                 460

Gly Gly Ser Gly Gly Asn Met Thr Trp Met Glu Trp Asp Arg Glu Ile
465                 470                 475                 480

Asn Asn Tyr Thr Ser Leu Ile His Ser Leu Ile Glu Glu Ser Gln Asn
                485                 490                 495

Gln Gln Glu Lys Asn Glu Gln Glu Leu Leu
            500                 505

<210>  83
<211>  36
<212>  PRT
<213>  Artificial

<220>
<223>  afp-2

<400>  83

Met Thr Trp Met Glu Trp Asp Arg Glu Ile Asn Gln Tyr Thr Ser Leu
1               5                   10                  15

Ile His Ser Leu Ile Glu Glu Ser Gln Asn Gln Gln Glu Lys Asn Glu
            20                  25                  30

Gln Glu Leu Leu
        35

## Description

The present invention relates to a conjugate of an antibody against CD4 and an antifusogenic peptide wherein two to eight antifusogenic peptides are each conjugated to one terminus of the heavy and/or light chains of an anti-CD4 antibody. The antifusogenic peptides can be different, similar or identical on the amino acid level.

### Background of the Invention

The infection of cells by the human immunodeficiency virus (HIV) is effected by a process in which the membrane of the cells to be infected and the viral membrane are fused. A general scheme for this process is proposed: The viral envelope glycoprotein complex (gp120/gp41) interacts with a cell surface receptor located on the membrane of the cell to be infected. The binding of gp120 to, e.g., the CD4 receptor in combination with a co-receptor such as CCR-5 or CXCR-4 causes a change in the conformation of the gp120/gp41 complex. In consequence of this conformational change the gp41 protein is able to insert into the membrane of the target cell. This insertion is the beginning of the membrane fusion process. It is known that the amino acid sequence of the gp41 protein varies in different HIV strains because of naturally occurring polymorphisms. But the same domain architecture can be recognized, more precisely, a fusion signal, two heptad repeat domains (HR1, HR2) and a transmembrane domain (in N- to C-terminal direction). It is suggested that the fusion (or fusogenic) domain is participating in the insertion into and the disintegration of the cell membrane. The HR regions are built up of multiple stretches comprising seven amino acids ("heptad") (see e.g. Shu, W., et al., Biochemistry 38 (1999) 5378-5385). Beside the heptads one or more leucine zipper-like motifs are present. This composition accounts for the formation of a coiled coil structure of gp41 proteins and just as well of peptides derived from these domains. Coiled coils are in general oligomers consisting of two or more interacting helices. Peptides with amino acid sequences deduced from the HR1 or the HR2 domain of gp41 are effective in vitro and in vivo inhibitors of HIV uptake into cells (for example peptides see e.g. US 5,464,933, US 5,656,480, US 6,258,782, US 6,348,568, or US 6,656,906). For example, T20 (also known as DP178, Fuzeon^{®}, a HR2 peptide), T651 (US 6,479,055), and T2635 (WO 2004/029074) are very potent inhibitors of HIV infection. It has been attempted to enhance the efficacy of HR2 derived peptides with, for example, amino acid substitutions or chemical crosslinking (Sia, S.K., et al., Proc. Natl. Acad. Sci. USA 99 (2002) 14664-14669; Otaka, A., et al., Angew. Chem. Int. Ed. 41 (2002) 2937-2940).

The conjugation of peptides to certain molecules can change their pharmacokinetic properties, e.g. the serum half-life of such peptide conjugates can be increased. Conjugations are reported, for example, for polyethylene glycol (PEG) and Interleukin-6 (EP 0 442 724), for PEG and Erythropoietin (WO 01/02017), for chimeric molecules comprising Endostatin and immunoglobulins (US 2005/008649), for secreted antibody based fusion proteins (US 2002/147311), for fusion polypeptides comprising albumin (US 2005/0100991; human serum albumin US 5,876,969), for PEGylated polypeptides (US 2005/0114037), and for interferon fusions. Also described in the state of the art are immunotoxins comprising Gelonin and an antibody (WO 94/26910), modified transferrin-antibody fusion proteins (US 2003/0226155), antibody-cytokine fusion proteins (US 2003/0049227), and fusion proteins consisting of a peptide with immuno-stimulatory, membrane transport, or homophilic activity and an antibody (US 2003/0103984). In WO 2004/085505 long acting biologically active conjugates consisting of biologically active compounds chemically linked to macromolecules, are reported.

T-lymphocytes are divided in CD4⁺- and CD8⁺-T-cells depending on the presence of the surface glycoprotein CD4 and CD8. The complexation of the CD4 surface glycoprotein can initiate e.g. T-cell proliferation or lymphokine release. CD4 is a membrane glycoprotein of 55 kDa which plays a central role in the initiation of T cell responses by interacting with MHC class 11 antigens on antigen presenting cells that present peptides to the antigen specific receptor on T cells. The effects are transmitted to the cytoplasm via a motif in the cytoplasmic domain with p56^{Ick} a tyrosine kinase involved in the activation of T lymphocytes (Brady, R.L. and Barclay, A.N., Curr. Top. Microbiol. Immunol. 205 (1996) 1-18; Rudd, C.E., et al., Proc. Natl. Acad. Sci. USA 85 (1988) 5190-5194; Weilette, A., et al., Eur. J. Immunol. 20 (1990) 1397-1400). The CD4 surface receptor is a target of the HI-virus required for entry into the cell. CD4⁺-lymophocites become non-functional upon infection with HIV.

In US 2006/0121480 a method for preventing infection of helper T and other target cells by HIV-1 by exposing target cells to a synergistic combination of at least one attachment inhibitor and at least one fusion inhibitor is reported. In WO 2004/103312 peptides useful as HIV fusion inhibitors are reported. A conjugate of an anti-CD4 molecule and an HIV-1 fusion inhibiting peptide is reported in WO 2001/43779. FC chimeric proteins with anti-HIV drugs are reported in WO 2004/108885.

### Summary of the Invention

The invention comprises a conjugate comprising two or more antifusogenic peptides and an anti-CD4 antibody (mAb CD4) characterized in that two to eight antifusogenic peptides are each conjugated to one terminus of the heavy and/or light chains of said anti-CD4 antibody (a number of eight antifusogenic peptides per mAb CD4 is only possible if the mAb CD4 comprises eight termini, i.e. is composed e.g. of two heavy chains and two light chains; if the mAb CD4 comprises a smaller number of C- and N-termini, e.g. as a scFv, the corresponding number of antifusogenic peptides possible at maximum in the conjugate is also reduced, i.e. it is reduced to less than eight).

In one embodiment the carboxy-terminal amino acid of an anti-CD4 antibody chain is conjugated to the amino-terminal amino acid of the antifusogenic peptide or the carboxy-terminal amino acid of the antifusogenic peptide is conjugated to the amino-terminal amino acid of the antibody chain, preferably by a peptide bond with or without an intermediate linker.

In another embodiment the conjugate is characterized by the general formula

mAb CD4 - [linker]ₘ - [antifusogenic peptide]ₙ

wherein m is independently for each antifusogenic peptide either 0 (i.e. a peptide bond between mAb CD4 and antifusogenic peptide) or 1 (i.e. a linker between mAb CD4 and antifusogenic peptide) and n is an integer of from 2 to 8, in one embodiment an even integer of from 2 to 8, preferably 2 or 4.

In one embodiment a preferred conjugate of a heavy and/or light chain of mAb CD4 and an antifusogenic peptide ("chain conjugate") is selected from the group consisting of (in N-terminal to C-terminal direction):
(1) [antifusogenic peptide] - [linker]ₘ - [heavy chain]
(2) [heavy chain] - [linker]ₘ - [antifusogenic peptide]
(3) [antifusogenic peptide] - [linker]ₘ - [heavy chain] - [antifusogenic peptide]
(4) [antifusogenic peptide] - [linker]ₘ - [light chain]
(5) [light chain] - [linker]ₘ - [antifusogenic peptide]
(6) [antifusogenic peptide] - [linker]ₘ - [light chain] - [antifusogenic peptide]
(7) [antifusogenic peptide] - [linker]ₘ - [heavy chain] - [linker]ₘ - [antifusogenic peptide]
(8) [antifusogenic peptide] - [linker]ₘ - [light chain] - [linker]ₘ - [antifusogenic peptide]
wherein the linker can be the same or different in (within and between) said chain conjugates, wherein m is an integer of 1 or 0, and m can be independently the same or different in (within and between) said chain conjugates. Left side of the peptide or mAb CD4 chain means N-terminus, right side means C-terminus. In (1) therefore the C-terminus of the antifusogenic peptide is linked by a peptide bond or a linker to the N-terminus of the heavy chain of mAb CD4.

In another embodiment the chain conjugates are assembled to conjugates according to the invention comprising a mAb CD4 (e.g. consisting of two light chains and two heavy chains including the constant Fc domains, a scFv fragment, or a Fab fragment).

Preferred chain conjugates are (2), (3), (4), and (7). Preferred conjugates according to the invention comprise 2x [mAb CD4 light chain] and 2x (2), 2 x [mAb CD4 light chain] and 2 x (3), or 2 x [mAb CD4 heavy chain] and 2 x (4), or 2 x [mAb CD4 light chain] and 2 x (7). The heavy and/or light chain comprises preferably a constant region.

In a further embodiment the conjugate according to the invention is characterized in comprising a variable heavy chain domain consisting of an immunoglobulin framework and a CDR3 region selected from the heavy chain CDR3 sequences of SEQ ID NO: 29, 30, or 31.

In still a further embodiment the conjugate is characterized in comprising a variable heavy chain domain consisting of an immunoglobulin framework and a CDR3 region selected from the CDR3 sequences of SEQ ID NO: 29, 30, or 31, a CDR2 region selected from the CDR2 sequences of SEQ ID NO: 26, 27, or 28, and a CDRI region selected from the CDR1 sequences of SEQ ID NO: 23, 24, or 25.

In another embodiment the conjugate is characterized in comprising a heavy chain variable domain wherein the heavy chain variable domain comprises SEQ ID NO: 08, or 10.

In one embodiment the conjugate is characterized in comprising a variable light chain domain consisting of an immunoglobulin framework and a CDR1 region selected from SEQ ID NO: 13, 14, 15, or 16, a CDR2 region selected from SEQ ID NO: 17, 18, or 19, and a CDR3 region selected from SEQ ID NO: 20, 21, or 22.

In another embodiment the conjugate is characterized in comprising as heavy chain CDRs the CDRs of SEQ ID NO: 07, and as light chain CDRs the CDRs of SEQ ID NO: 01, as heavy chain CDRs the CDRs of SEQ ID NO: 08, and as light chain CDRs the CDRs of SEQ ID NO: 02, as heavy chain CDRs the CDRs of SEQ ID NO: 10, and as light chain CDRs the CDRs of SEQ ID NO: 04, as heavy chain CDRs the CDRs of SEQ ID NO: 11, and as light chain CDRs the CDRs of SEQ ID NO: 05, or as heavy chain CDRs the CDRs of SEQ ID NO: 12, and as light chain CDRs the CDRs of SEQ ID NO: 06.

In one embodiment the conjugate is characterized in comprising a variable heavy and light chain domain independently selected from:
a) the heavy chain variable domain defined by amino acid sequence SEQ ID NO: 07, and the light chain variable domain defined by amino acid sequence SEQ ID NO: 01;
b) the heavy chain variable domain defined by amino acid sequence SEQ ID NO: 08, and the light chain variable domain defined by amino acid sequence SEQ ID NO: 02;
c) the heavy chain variable domain defined by amino acid sequence SEQ ID NO: 10, and the light chain variable domain defined by amino acid sequence SEQ ID NO: 04;
d) the heavy chain variable domain defined by amino acid sequence SEQ ID NO: 11, and the light chain variable domain defined by amino acid sequence SEQ I D NO: 05,
e) the heavy chain variable domain defined by amino acid sequence SEQ ID NO: 12, and the light chain variable domain defined by amino acid sequence SEQ I D NO: 06.

In another embodiment the conjugate is characterized in comprising the heavy chain variable domain defined by amino acid sequence SEQ ID NO: 07, and the light chain variable domain defined by amino acid sequence SEQ ID NO: 01; or the heavy chain variable domain defined by amino acid sequence SEQ ID NO: 08, and the light chain variable domain defined by amino acid sequence SEQ ID NO: 02; or the heavy chain variable domain defined by amino acid sequence SEQ ID NO: 10, and the light chain variable domain defined by amino acid sequence SEQ ID NO: 04; the heavy chain variable domain defined by amino acid sequence SEQ ID NO: 11, and the light chain variable domain defined by amino acid sequence SEQ ID NO: 05, or the heavy chain variable domain defined by amino acid sequence SEQ ID NO: 12, and the light chain variable domain defined by amino acid sequence SEQ ID NO: 06; a linker selected from the amino acids glycine (G) and asparagine (N), the tripeptide GST, and the SEQ ID NO: 46 to 76; and an antifusogenic peptide selected from the antifusogenic peptides of SEQ ID NO: 35 to 44.

In one embodiment the conjugate is characterized in comprising an antifusogenic peptide selected from the antifusogenic peptides C34, T20, T1249, T651, T2635, N36, DP107, or afp-1.

In another embodiment the conjugate is characterized in comprising an antifusogenic peptide at each C-terminus of the heavy chains or at each N-terminus of the light chains (two antifusogenic peptides). In one embodiment the conjugate is characterized in that it comprises an antifusogenic peptide at each C-terminus of the heavy chains and at each N-terminus of the light chains (four antifusogenic peptides). In a further embodiment the conjugate is characterized in that it comprises an antifusogenic peptide at the two C-termini of the heavy chains and at the two N-termini of the light chains (four antifusogenic peptides)

In one embodiment the conjugate is characterized in comprising two light chain variable domains of SEQ ID NO: 01, two conjugates of type (2) each comprising a heavy chain variable domain of SEQ ID NO: 07, a linker of SEQ ID NO: 75, and an antifusogenic peptide of SEQ ID NO: 42, in comprising two light chain variable domains of SEQ ID NO: 02, two conjugates of type (2) each comprising a heavy chain variable domain of SEQ ID NO: 08, a linker of SEQ ID NO: 75, and an antifusogenic peptide of SEQ ID NO: 42, in comprising two light chain variable domains of SEQ ID NO: 04, two conjugates of type (2) each comprising a heavy chain variable domain of SEQ ID NO: 10, a linker of SEQ ID NO: 75, and an antifusogenic peptide of SEQ ID NO: 42, in comprising two light chain variable domains of SEQ ID NO: 05, two conjugates of type (2) each comprising a heavy chain variable domain of SEQ ID NO: 11, a linker of SEQ ID NO: 75, and an antifusogenic peptide of SEQ ID NO: 42, or in comprising two light chain variable domains of SEQ ID NO: 06, two conjugates of type (2) each comprising a heavy chain variable domain of SEQ ID NO: 12, a linker of SEQ ID NO: 75, and an antifusogenic peptide of SEQ ID NO: 42.

In one embodiment the conjugate is characterized in that said anti-CD4 antibody is of IgG1 subclass or of IgG4 subclass. It is also in one embodiment that said anti-CD4 antibody is of IgG4 or IgG 1 or IgG2 subclass, with a mutation in amino acid position S228, L234, L235, and/or D265, and/or contains the PVA236 mutation. In another embodiment the conjugate is characterized in that said anti-CD4 antibody of IgG4 subclass has a S228P mutation and said anti-CD4 antibody of IgG1 subclass has L234A and L235A mutations.

The invention comprises a method for the production of a conjugate according to the invention, characterized in that the method comprises
a) cultivating a cell containing one or more plasmids each containing one or more nucleic acid molecules encoding a conjugate according to the invention under conditions suitable for the expression of the conjugate,
b) recovering the conjugate from the cell or the supernatant.

In one embodiment the genes encoding the light and heavy chains of mAb CD4 with or without linked antifusogenic peptide are located on the same expression vector or on different expression vectors.

The invention also comprises a pharmaceutical composition, containing a conjugate according to the invention, together with a pharmaceutically acceptable excipient or carrier.

The invention further comprises the use of a conjugate according to the invention for the manufacture of a medicament for the treatment of viral infections. In one embodiment the use is characterized in that the viral infection is a HIV infection.

The invention comprises the use of a conjugate according to the invention for the treatment of a patient in need of an antiviral treatment, preferably an anti HIV treatment.

### Description of the Invention

The current invention reports a conjugate comprising two or more antifusogenic peptides and an anti-CD4 antibody (mAb CD4) characterized in that two to eight antifusogenic peptides are each conjugated to one terminus of the heavy and/or light chains of said anti-CD4 antibody. A number of eight antifusogenic peptides per mAb CD4 is only possible if the mAb CD4 comprises eight termini, i.e. is composed e.g. of two heavy chains and two light chains. If the mAb CD4 comprises a smaller number of C- and N-termini, e.g. as a scFv, the corresponding number of antifusogenic peptides possible at maximum in the conjugate is also reduced, i.e. it is reduced to less than eight.

An "antifusogenic peptide" is a peptide which inhibits events associated with membrane fusion or the membrane fusion event itself, including, among other things, the inhibition of infection of uninfected cells by a virus due to membrane fusion. These antifusogenic peptides are in one embodiment linear peptides. For example, they can be derived from the gp41 ectodomain, e.g. such as DP107, DP178. Examples of such peptides can be found in US 5,464,933, US 5,656,480, US 6,013,263, US 6,017,536, US 6,020,459, US 6,093,794, US 6,060,065, US 6,258,782, US 6,348,568, US 6,479,055, US 6,656,906, WO 1996/19495, WO 1996/40191, WO 1999/59615, WO 2000/69902, and WO 2005/067960. For example, the amino acid sequences of such peptides comprise or can be selected from the group of SEQ ID NO: 1 to 10 of US 5,464,933; SEQ ID NO: 1 to 15 of US 5,656,480; SEQ ID NO: 1 to 10 and 16 to 83 of US 6,013,263; SEQ ID NO: 1 to 10, 20 to 83 and 139 to 149 of US 6,017,536; SEQ ID NO: 1 to 10, 17 to 83 and 210 to 214 of US 6,093,794; SEQ ID NO: 1 to 10, 16 to 83 and 210 to 211 of US 6,060,065; SEQ ID NO: 1286 and 1310 of US 6,258,782; SEQ ID NO: 1129, 1278-1309, 1311 and 1433 of US 6,348,568; SEQ ID NO: 1 to 10 and 210 to 238 of US 6,479,055; SEQ ID NO: 1 to 171, 173 to 216, 218 to 219, 222 to 228, 231, 233 to 366, 372 to 398, 400 to 456, 458 to 498, 500 to 570, 572 to 620, 622 to 651, 653 to 736, 739 to 785, 787 to 811, 813 to 823, 825, 827 to 863, 865 to 875, 877 to 883, 885, 887 to 890, 892 to 981, 986 to 999, 1001 to 1003, 1006 to 1018, 1022 to 1024, 1026 to 1028, 1030 to 1032, 1037 to 1076, 1078 to 1.079, 1082 to 1117, 1120 to 1176, 1179 to 1213, 1218 to 1223, 1227 to 1237, 1244 to 1245, 1256 to 1268, 1271 to 1275, 1277, 1345 to 1348, 1350 to 1362, 1364, 1366, 1368, 1370, 1372, 1374 to 1376, 1378 to 1379, 1381 to 1385, 1412 to 1417, 1421 to 1426, 1428 to 1430, 1432, 1439 to 1542, 1670 to 1682, 1684 to 1709, 1712 to 1719, 1721 to 1753, 1755 to 1757 of US 6,656,906; or SEQ ID NO: 5 to 95 of WO 2005/067960. In one embodiment the antifusogenic peptide has an amino acid sequence comprising of from 5 to 100 amino acids, preferably of from 10 to 75 amino acids, and more preferred of from 15 to 50 amino acids. In a preferred embodiment antifusogenic peptides are C-34, T-20, T-1249, T-651, T-2635, N-36, (Root, M.J., et al., Curr. Pharm. Des. 10 (2004) 1805-1825) and DP-107 (Wild, C., et al., Proc. Natl. Acad. Sci. USA 91 (1994) 12676-12680). In one embodiment the conjugate according to the invention comprises one or more antifusogenic peptides and an anti-CD4 antibody (mAb CD4) wherein i) said antifusogenic peptides are linear peptides with an amino acid sequence of from 5 to 100 amino acids, and ii) one to eight antifusogenic peptides are each conjugated to one terminus of the heavy and/or light chains of said anti-CD4 antibody. In another embodiment the conjugate according to the invention comprises one or more antifusogenic peptides and an anti-CD4 antibody (mAb CD4) wherein i) said antifusogenic peptides are derived from the gp41 ectodomain, and ii) one to eight antifusogenic peptides are each conjugated to one terminus of the heavy and/or light chains of said anti-CD4 antibody. The term "gp41 ectodomain" denotes the amino acid sequence starting with amino acid position 561 and ending with amino acid position 620 of HIV-1 gp160 or starting with amino acid position 50 and ending with amino acid position 109 of HIV-1 gp41 (SEQ ID NO: 66) (see also e.g. Bar, S. and Alizon, M. J. Virol. 78 (2004) 811-820).

The term "antibody" encompasses the various forms of antibody structures including whole antibodies and antibody fragments. The antibody according to the invention is in one embodiment a human antibody, a humanized antibody, a chimeric antibody, or a T cell antigen depleted antibody (see e.g. WO 98/33523, WO 98/52976, and WO 00/34317). Genetic engineering of antibodies is e.g. described in Morrison, S.L., et al., Proc. Natl. Acad Sci. USA 81 (1984) 6851-6855; US 5,202,238 and US 5,204,244; Riechmann, L., et al., Nature 332 (1988) 323-327; Neuberger, M.S., et al., Nature 314 (1985) 268-270; Lonberg, N., Nat. Biotechnol. 23 (2005) 1117-1125.

"Antibody fragments" comprise a portion of a full length anti-CD4 antibody, preferably the variable domains thereof or at least the antigen binding portion thereof. Examples of antibody fragments are e.g. single-chain antibody molecules (scFv), Fab, F(ab)₂ fragments, and the like as long as they retain the binding characteristics of an anti-CD4 antibody. ScFv antibodies are, e.g., described in Huston, J.S., Methods in Enzymol. 203 (1991) 46-88. Huston also describes linkers and methods for linking of polypeptides useful for the present invention.

"CD4" means human CD4 as described, e.g., in Brady, R.L. and Barclay, A.N., Curr. Top. Microbiol. Immunol. 205 (1996) 1-18 and SwissProt P01730. The terms "antibody binding to CD4", "anti-CD4 antibody", or "mAb CD4", which are used interchangeably within this application, denote an antibody specifically binding to CD4 and preferably inhibiting HIV fusion with a target cell. Binding can be tested

in a cell based in vitro ELISA assay (using CD4 expressing CHO cells). Binding is found, if the antibody in question causes an S/N (signal/noise) ratio of 5 or more, preferably 10 or more at an antibody concentration of 100 ng/ml. The term "inhibiting HIV fusion with a target cell" refers to inhibiting HIV fusion with a target cell measured in an assay comprising contacting said target cell (e.g. PBMC) with the virus in the presence of the antibody in question in a concentration effective to inhibit membrane fusion between the virus and said cell and measuring, e.g., luciferase reporter gene activity or the HIV p24 antigen concentration. The term "membrane fusion" refers to fusion between a first cell expressing CD4 polypeptides and a second cell or virus expressing an HIV env protein. Membrane fusion is determined by genetically engineered cells and/or viruses by a reporter gene assay (e.g. by luciferase reporter gene assay).

The term "chimeric" as used within the current application denotes that the antibody or antibody domain comprises amino acid sequences derived from a an antibody from a non-human animal as well as amino acid sequences derived from an antibody of human origin.

Preferred anti-CD4 antibodies are mentioned in e.g. Reimann, K.A., et al., Aids Res. Human Retrovir. 13 (1997) 933-943, EP 0 512 112, US 5,871,732, EP 0 840 618, EP 0 854 885, EP 1 266 965, US 2006/0051346, WO 97/46697, WO 01/43779, US 6,136,310, WO91/009966. Especially preferred anti-CD4 antibodies are described in US 5,871,732, and Reimann, K.A., et al., Aids Res. Human Retrovir. 13 (1997) 933-943, and WO 91/009966. An especially preferred anti-CD4 antibody is characterized in that it is a non-immunosuppressive or non-depleting antibody when administered to humans and also does not block binding of HIV gp120 to human CD4. In one embodiment a preferred anti-CD4 antibody is further characterized in that the antibody comprises a variable heavy chain domain consisting of an immunoglobulin framework and a CDR3 region selected from the heavy chain CDR3 sequences of SEQ ID NO: 29, 30, or 31. In a further preferred embodiment the antibody comprises a variable heavy chain region consisting of an immunoglobulin framework and a CDR3 region selected from the CDR3 sequences of SEQ ID NO: 29, or 30, or 31, a CDR2 region selected from the CDR2 sequences of SEQ ID NO: 26, or 27, and a CDR1 region selected from the CDR1 sequences of SEQ ID NO: 23, or 24. In a preferred embodiment heavy chain variable domains are shown in SEQ ID NO: 08, 10, or 12. In a preferred embodiment the anti-CD4 antibody comprises in addition a variable light chain domain consisting of an immunoglobulin framework and a CDR1 region selected from the CDR1 sequences of SEQ ID NO: 13, 14, or 15, a CDR2 region selected from the CDR2 sequences of SEQ ID NO: 17, or 18, and a CDR3 region selected from the CDR3 sequences of SEQ 10 NO: 20, or 21. The anti-CD4 antibody is in one embodiment characterized in containing as heavy chain CDRs the CDRs of SEQ ID NO: 08, and as light chain CDRs the CDRs of SEQ ID NO: 02, as heavy chain CDRs the CDRs of SEQ ID NO: 10, and as light chain CDRs the CDRs of SEQ ID NO: 04, as heavy chain CDRs the CDRs of SEQ ID NO: 11, and as light chain CDRs the CDRs of SEQ ID NO: 05, or as heavy chain CDRs the CDRs of SEQ ID NO: 12, and as light chain CDRs the CDRs of SEQ ID NO: 06.

CDR sequences can be determined according to the standard definition of Kabat, E.A., et al., Sequences of Proteins of Immunological Interest, 5th ed., Public Health Service, National Institutes of Health, Bethesda, MD (1991). CDRs of SEQ ID NO: 01 to SEQ ID NO: 12 are shown in SEQ ID NO: 13 to 31.

The anti-CD4 antibody comprises in one embodiment a variable heavy and light chain domain independently selected from the group consisting of
a) the heavy chain variable domain defined by amino acid sequence SEQ ID NO: 08, and the light chain variable domain defined by SEQ ID NO: 02;
b) the heavy chain variable domain defined by amino acid sequence SEQ ID NO: 10, and the light chain variable domain defined by SEQ ID NO: 04;
c) the heavy chain variable domain defined by amino acid sequence SEQ ID NO: 11, and the light chain variable domain defined by SEQ ID NO: 05;
d) the heavy chain variable domain defined by amino acid sequence SEQ ID NO: 12, and the light chain variable domain defined by SEQ I D NO: 06.

The antibody used in the conjugate according to the invention is in one embodiment characterized in that the constant domains are of human origin. Such constant domains are well known in the state of the art and, e.g., described by Kabat (see e.g. Johnson, G., and Wu, T.T., Nucleic Acids Res. 28 (2000) 214-218). For example, in one embodiment a useful human IgG1 heavy chain constant region (C_{H}1-Hinge-C_{H}2-C_{H}3) comprises an amino acid sequence independently selected from the group consisting of SEQ ID NO: 32, 33. For example, a useful human kappa (κ) light chain constant domain comprises an amino acid sequence of a kappa light chain constant domain (κ light chain constant domain, C_{L}) of SEQ ID NO: 34. In another embodiment the antibody's variable domains are of mouse origin and comprise the antibody variable domain sequence frame of a mouse antibody according to Kabat (see e.g. Johnson, G., and Wu, T.T., Nucleic Acids Res. 28 (2000) 214-218). In another embodiment the antibody's variable domains are of mouse origin and have been humanized.

A preferred anti-CD4 antibody shows a binding to the same epitope(s) of CD4 as does an antibody with variable domains 1 (US 5,871,732) or variable domains 2 (Reimann, K.A., et al., Aids Res. Human Retrovir. 13 (1997) 933-943) or is inhibited in binding to CD4 by antibodies with variable domains 1 or variable domains 2 due to steric hindrance of binding or competitive binding. Epitope binding is investigated by using alanine scanning according to the method described by Olson, W.C., et al. (J. Virol. 73 (1999) 4145-4155) for epitope mapping. A signal reduction of 75 % or more shows that the mutated amino acid(s) contribute to the epitope recognized by said antibody. Binding of the antibody to the same epitope is found, if the amino acids contributing to the epitope are recognized by the investigated antibody and antibodies with variable domains 1 or variable domains 2.

The term "epitope" denotes a protein determinant capable of specific binding to an antibody. Epitopes usually consist of chemically active surface groupings of molecules such as amino acids or sugar side chains and usually have specific three dimensional structural characteristics, as well as specific charge characteristics. Conformational and non-conformational epitopes are distinguished in that the binding to the former but not the latter is lost in the presence of denaturing solvents.

The term "variable domain" (variable domain of a light chain (V_{L}), variable domain of a heavy chain (V_{H})) as used herein denotes the individual domains of the pair of light and heavy chains of an antibody which are directly involved in the binding of the target antigen by the antibody. The variable domains are generally the N-terminal domains of a pair of light and heavy chains. The variable domains of the light and heavy chain have the same general structure, i.e. they possess an "immunoglobulin framework", and each domain comprises four "framework regions" (FR), whose sequences are widely conserved, connected by three "hypervariable regions" (or "complementarity determining regions", CDRs). The framework regions adopt a β-sheet conformation and the CDRs may form loops connecting the β-sheet structure. The CDRs in each chain are held in their three-dimensional structure by the framework regions and form together with the CDRs from the other chain the antigen binding site. The antibody heavy and light chain CDR3 regions play a particularly important role in the binding specificity/affinity of the antibodies according to the invention and therefore provide a further object of the invention.

The terms "antigen-binding portion of an antibody" or "antigen-binding site of an antibody" when used herein refer to the amino acid residues of an antibody which are responsible for antigen binding. The antigen-binding site of an antibody comprises amino acid residues from the "complementarity determining regions" or "CDRs". "Framework" or "FR" regions are those variable domain regions other than the hypervariable region residues as herein defined. Therefore, the light and heavy chain variable domains of an antibody comprise from N- to C-terminus the regions FR1, CDR1, FR2, CDR2, FR3, CDR3, and FR4 (immunoglobulin framework). Especially, the CDR3 region of the heavy chain is the region which contributes most to antigen binding and defines the antibody. In one embodiment the antibody according to the invention is characterized by comprising in its heavy chain variable domain the CDR3 sequence of SEQ ID NO: 29, or SEQ ID NO: 30. Complementarity determining (CDR) and framework (FR) regions are determined according to the standard definition of Kabat, E.A., et al., Sequences of Proteins of Immunological Interest, 5th ed., Public Health Service, National Institutes of Health, Bethesda, MD (1991).

The "Fc part" of an anti-CD4 antibody is not involved directly in binding to CD4, but exhibit various effector functions. Depending on the amino acid sequence of the constant region of the heavy chains, antibodies (immunoglobulins) are divided in the classes: IgA, IgD, IgE, IgG, and IgM. Some of these classes are further divided into subclasses (isotypes), i.e. IgG in IgG1, IgG2, IgG3, and IgG4, or IgA in IgA1 and IgA2. According to the immunoglobulin class to which an antibody belongs are the heavy chain constant regions of immunoglobulins are denoted α (alpha, IgA), δ (delta, IgD), ε (epsilon, IgE), γ (gamma, IgG), and µ (miu, IgM), respectively. The antibodies according to the invention belong in one embodiment to the IgG class. An "Fc part of an antibody" is a term well known to the skilled artisan and defined on basis of the papain cleavage of antibodies. The antibodies according to the invention contain as Fc part a human Fc part or an Fc part derived from human origin. In a further embodiment of the invention the Fc part is either an Fc part of a human antibody of the subclass IgG4 or an Fc part of a human antibody of the subclass IgG1, IgG2, or IgG3, which is modified in such a way that no Fcγ receptor (e.g. FcγRIIIa) binding and/or no C1q binding as defined below can be detected. In one embodiment the Fc part is a human Fc part and preferably either from human IgG4 subclass or a mutated Fc part from human IgG1 subclass. In another embodiment the FC part is an Fc part from human IgG1 subclass with mutations L234A and L235A. In a further embodiment the Fc part is an Fc part of SEQ ID NO: 32, SEQ ID NO: 33, SEQ ID NO: 32 with mutations L234A and L235A, SEQ ID NO: 33 with mutation S228P. While IgG4 shows reduced Fcγ receptor (FcγRIIIa) binding, antibodies of other IgG subclasses show strong binding. However Pro238, Asp265, Asp270, Asn297 (loss of Fc carbohydrate), Pro329, Leu234, Leu235, Gly236, Gly237, Ile253, Ser254, Lys288, Thr307, Gln311, Asn434, or/and His435 are residues which, if altered, provide also reduced Fcγ receptor binding (Shields, R.L., et al., J. Biol. Chem. 276 (2001) 6591-6604; Lund, J., et al., FASEB J. 9 (1995) 115-119; Morgan, A., et al., Immunology 86 (1995) 319-324; EP 0 307 434). In one embodiment an antibody according to the invention is in regard to Fcγ receptor binding of IgG4 subclass or of IgG1 or IgG2 subclass, with a mutation in L234, L235, and/or D265, and/or contains the PVA236 mutation. In a preferred embodiment the mutations are S228P, L234A, L235A, L235E, and/or PVA236 (PVA236 means that the amino acid sequence ELLG (given in one letter amino acid code) from amino acid position 233 to 236 of IgG1 or EFLG of IgG4 is replaced by PVA), preferably the mutations S228P of IgG4, and L234A and L235A of IgG1. The Fc part of an antibody is directly involved in ADCC (antibody-dependent cell-mediated cytotoxicity) and CDC (complement-dependent cytotoxicity). Complement activation (CDC) is initiated by binding of complement factor C1q to the Fc part of most IgG antibody subclasses. Binding of C1q to an antibody is caused by defined protein-protein interactions at the so called binding site. Such Fc part binding sites are known in the state of the art and described e.g. by Lukas, T.J., et al., J. Immunol. 127 (1981) 2555-2560; Brunhouse, R. and Cebra, J.J., Mol. Immunol. 16 (1979) 907-917; Burton, D.R., et al., Nature 288 (1980) 338-344; Thommesen, J.E., et al., Mol. Immunol. 37 (2000) 995-1004; Idusogie, E.E., et al., J. Immunol. 164 (2000) 4178-4184; Hezareh, M., et al., J. Virol. 75 (2001) 12161-12168; Morgan, A., et al., Immunology 86 (1995) 319-324; and EP 0 307 434. Such Fc part binding sites are, among other things, characterized by the amino acids L234, L235, D270, N297, E318, K320, K322, P331, and P329 (numbering according to EU index of Kabat). Antibodies of subclass IgGl, IgG2, and IgG3 usually show complement activation including C1q and C3 binding, whereas IgG4 does not activate the complement system and does not bind C1q and/or C3. An anti-CD4 antibody which does not bind Fcγ receptor and/or complement factor C1q does not elicit antibody-dependent cellular cytotoxicity (ADCC) and/or complement dependent cytotoxicity (CDC). In one embodiment this antibody is characterized in that it binds CD4, contains an Fc part derived from human origin, and does not bind Fcγ receptors and/or complement factor C1q. In a preferred embodiment this antibody is a human, or a humanized, or a T-cell antigen depleted antibody. C1q binding can be measured according to Idusogie, E.E., et al., J. Immunol. 164 (2000) 4178-4184. No "C1q binding" is found if in such an assay the optical density (OD) at 492-405 nm is for the antibody in question lower than 15 % of the value for human C1q binding of the unmodified wild-type antibody Fc part at an antibody concentration of 8 µg/ml. ADCC can be measured as binding of the antibody to human FcγRIIIa on human NK cells. Binding is determined at an antibody concentration of 20 µg/ml. "No Fcγ receptor binding" or "no ADCC" means a binding of up to 30 % to human FcγRIIIa on human NK cells at an antibody concentration of 20 µg/ml compared to the binding of the same antibody as human IgG1 (SEQ ID NO: 32).

An antibody used in a conjugate according to the invention include, in addition, such antibodies having "conservative sequence modifications" (variant antibodies), which are amino acid sequence modifications which do not affect or alter the above-mentioned characteristics of the antibody according to the invention. Modifications can be introduced by standard techniques known in the art, such as site-directed mutagenesis and PCR-mediated mutagenesis. Conservative amino acid substitutions include ones in which the amino acid residue is replaced with an amino acid residue having a similar side chain. Families of amino acid residues having similar side chains have been defined in the art. These families include amino acids with basic side chains (e.g. lysine, arginine, histidine), acidic side chains (e.g. aspartic acid, glutamic acid), uncharged polar side chains (e.g. glycine, asparagine, glutamine, serine, threonine, tyrosine, cysteine, tryptophan), non-polar side chains (e.g. alanine, valine, leucine, isoleucine, proline, phenylalanine, methionine), beta-branched side chains (e.g. threonine, valine, isoleucine), and aromatic side chains (e.g. tyrosine, phenylalanine, tryptophan, histidine). Thus, a predicted nonessential amino acid residue in a human anti-CD4 antibody can in one embodiment be replaced with another amino acid residue from the same side chain family. A "variant" anti-CD4 antibody, refers therefore herein to a molecule which differs in amino acid sequence from a "parent" anti-CD4 antibody's amino acid sequence in one embodiment by up to ten, in another embodiment from about two to about five, additions, deletions, and/or substitutions in one or more of the variable domain regions of the parent antibody outside the heavy chain CDR3 region. Each other heavy chain CDR region comprises at maximum one single amino acid addition, deletion, and/or substitution. The invention comprises a method of modifying the CDR amino acid sequence of a parent antibody binding to CD4, characterized in selecting a heavy chain variable domain from the heavy chain variable domains of SEQ ID NO: 08, or 10, and/or a light chain variable domain from the light chain variable domains of SEQ ID NO: 02, or 04, providing a nucleic acid encoding said initial variable domain amino acid sequence, modifying said nucleic acid in that one amino acid is modified in heavy chain CDR1, one amino acid is modified in heavy chain CDR2, 1-3 amino acid are modified in light chain CDR1, 1-3 amino acids are modified in light chain CDR2, and/or 1-3 amino acids are modified in light chain CDR3, expressing and incorporating said modified variable domain(s) amino acid sequence in an antibody structure, measuring whether said antibody binds to CD4 and selecting said modified variable domain(s)/CDR(s) if the antibody binds to CD4. In one embodiment such modifications are conservative sequence modifications. Amino acid sequence modifications can be performed by mutagenesis based on molecular modeling as described by Riechmann, L., et al., Nature 332 (1988) 323-327, and Queen, C., et al., Proc. Natl. Acad. Sci. USA 86 (1989) 10029-10033.

The term "linker" or "peptidic linker" as used within this application denotes peptide linkers of natural and/or synthetic origin. They consist of a linear amino acid chain wherein the 20 naturally occurring amino acids are the monomeric building blocks. The chain has a length of from 1 to 50 amino acids, preferred between 1 and 28 amino acids, especially preferred between 3 and 25 amino acids. The linker may contain repetitive amino acid sequences or sequences of naturally occurring polypeptides, such as polypeptides with a hinge-function. The linker has the function to ensure that a peptide conjugated to an anti-CD4 antibody can perform its biological activity by allowing the peptide to fold correctly and to be presented properly. In one embodiment the linker is a "synthetic peptidic linker" that is designated to be rich in glycine, glutamine, and/or serine residues. These residues are arranged e.g. in small repetitive units of up to five amino acids, such as GGGGS, QQQQG, or SSSSG. This small repetitive unit may be repeated for two to five times to form a multimeric unit. At the amino- and/or carboxy-terminal ends of the multimeric unit up to six additional arbitrary, naturally occurring amino acids may be added. Other synthetic peptidic linkers are composed of a single amino acid, that is repeated between 10 to 20 times and may comprise at the amino- and/or carboxy-terminal end up to six additional arbitrary, naturally occurring amino acids, such as e.g. serine in the linker GSSSSSSSSSSSSSSSG (SEQ ID NO: 64). Preferred linkers are shown in Table 2. In one embodiment the linker is selected from [GQ₄]₃GNN (SEQ ID NO: 50), LSLSPGK (SEQ ID NO: 46), LSPNRGEC (SEQ ID NO: 47), LSLSGG (SEQ ID NO: 71), LSLSPGG (SEQ ID NO: 72), G₃[SG₄]₂SG (SEQ ID NO: 75), or G₃[SG₄]₂SG₂ (SEQ ID NO: 76). All peptidic linkers can be encoded by a nucleic acid molecule and therefore can be recombinantly expressed. As the linkers are themselves peptides, the antifusogenic peptide is connected to the linker via a peptide bond that is formed between two amino acids. The peptidic linker is introduced between the antifusogenic peptide and the anti-CD4 antibody chain to which the antifusogenic peptide is to be conjugated. Therefore two or three, respectively, possible sequences (in amino- to carboxy-terminal direction) exist: a) antifusogenic peptide - peptidic linker - anti-CD4 antibody polypeptide chain, or b) anti-CD4 antibody polypeptide chain - peptidic linker - antifusogenic peptide, or c) antifusogenic peptide - peptidic linker - anti-CD4 antibody polypeptide chain - peptidic linker - antifusogenic peptide.

In one embodiment of the invention the conjugate is characterized in comprising i) the heavy chain variable domain defined by amino acid sequence SEQ ID NO: 08, and the light chain variable domain defined by SEQ ID NO: 02; or the heavy chain variable domain defined by amino acid sequence SEQ ID NO: 10, and the light chain variable domain defined by SEQ ID NO: 04; or the heavy chain variable domain defined by amino acid sequence SEQ ID NO: 11, and the light chain variable domain defined by SEQ ID NO: 05; or the heavy chain variable domain defined by amino acid sequence SEQ ID NO: 12, and the light chain variable domain defined by SEQ ID NO: 06; ii) a linker selected from the amino acids glycine (G) and asparagine (N), the tripeptide GST, and SEQ ID NO: 46 to 76 or 80 or 81; and iii) an antifusogenic peptide selected from the antifusogenic peptides of SEQ ID NO: 35 to 44 or 83. A further aspect of the current invention is the antifusogenic peptide afp-1 of SEQ ID NO: 42. In one embodiment the antifusogenic peptide is of SEQ ID NO: 36, 38, 39, or 42 or 83. In another embodiment the linker is of SEQ ID NO: 46, 47, 50, 64, 71, 72, 75, or 76.

In one embodiment a conjugate of a heavy and/or light chain of mAb CD4 and an antifusogenic peptide(s) ("chain conjugate") is selected from the chain conjugates of the order (1) [antifusogenic peptide] - [linker]ₘ - [heavy chain], (2) [heavy chain] - [linker]ₘ - [antifusogenic peptide], (3) [antifusogenic peptide] - [linker]ₘ - [heavy chain] - [antifusogenic peptide], (4) [antifusogenic peptide] - [linker]ₘ - [light chain], (5) [light chain] - [linker]ₘ - [antifusogenic peptide], (6) [antifusogenic peptide] - [linker]ₘ - [light chain] - [antifusogenic peptide], (7) [antifusogenic peptide] - [linker]ₘ - [heavy chain] - [linker]ₘ - [antifusogenic peptide], (8) [antifusogenic peptide] - [linker]ₘ - [light chain] - [linker]ₘ - [antifusogenic peptide], wherein the linker can be the same or different both within and between said chain conjugates, wherein m is an integer of 1 or 0, and m can be independently the same or different both within and between said chain conjugates. For example, in a conjugate comprising a chain conjugate (7) and a mAb CD4 light chain the two linkers in chain conjugate (7) can be the same, i.e. have the same amino acid sequence and length, or can be different, i.e. have different amino acid sequences and/or lengths, or one or both can be absent. For example, in a conjugate comprising chain conjugates (2) and (4) the linker contained in chain conjugate (2) and the linker contained in chain conjugate (4) can be the same, i.e. have the same amino acid sequence and length, or can be different, i.e. have different amino acid sequences and/or lengths, or one or both can be absent. In the chain conjugates the linker(s) can be present (m=1) or absent (m=0). Preferred chain conjugates are the chain conjugates (2), (3), (4), and (7). One embodiment of the current invention is a conjugate comprising 2 x [mAb CD4 light chain] and 2 x chain conjugate (2). This conjugate comprises two not conjugate anti-CD4 antibody light chains and two anti-CD4 antibody heavy chains each conjugated via its C-terminus to the N-terminus of a single antifusogenic peptide, optionally with an intermediate linker. Another embodiment of the current invention is a conjugate comprising two mAb CD4 light chains and two chain conjugates (3). Still another embodiment is a conjugate comprising two mAb CD4 heavy chains and two chain conjugates (4). A further embodiment of the current invention is a conjugate comprising two mAb CD4 light chains and two chain conjugates (7). The heavy and/or light chain comprises preferably a constant region (Fc).

The invention further provides a method for the manufacture of a pharmaceutical composition comprising an effective amount of a conjugate according to the invention together with a pharmaceutically acceptable carrier and the use of the conjugate according to the invention for such a method.

The invention further provides the use of a conjugate according to the invention in an effective amount for the manufacture of a pharmaceutical agent, preferably together with a pharmaceutically acceptable carrier, for the treatment of a patient suffering from AIDS.

The term "amino acid" as used within this application denotes the group of carboxy α-amino acids, which directly or in form of a precursor can be encoded by a nucleic acid. The individual amino acids are encoded by nucleic acids consisting of three nucleotides, so called codons or base-triplets. Each amino acid is encoded by at least one codon. This is known as "degeneration of the genetic code". The term "amino acid" as used within this application denotes the naturally occurring carboxy α-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

Methods and techniques known to a person skilled in the art, which are useful for carrying out the current invention, are described e.g. in Ausubel, F.M., ed., Current Protocols in Molecular Biology, Volumes I to III (1997), Wiley and Sons; Sambrook et al., Molecular Cloning: A Laboratory Manual, Second Edition, Cold Spring Harbor Laboratory Press, Cold Spring Harbor, N.Y. (1989).

In the conjugates according to the invention in one embodiment the carboxy-terminal amino acid of an anti-CD4 antibody chain is conjugated via a peptide bond to the amino-terminal amino acid of the antifusogenic peptide or the carboxy-terminal amino acid of the antifusogenic peptide is conjugated via a peptide bond to the amino-terminal amino acid of an anti-CD4 antibody chain. In one embodiment an intermediate linker is present between the antifusogenic peptide and the anti-CD4 antibody chain. Thus, the conjugate according to the invention is characterized by the general formula

mAb CD4 - [linker]ₘ- [antifusogenic peptide]ₙ

wherein m is independently for each antifusogenic peptide either 0 (i.e. a direct peptide bond between mAb CD4 and the antifusogenic peptide) or 1 (i.e. a linker is present between mAb CD4 and antifusogenic peptide) and n is an integer of from 2 to 8. In one embodiment n is an integer of from 2 to 8. In another embodiment n is an even integer of from 2 to 8. In another embodiment n is an integer of from 2 to 4. In another embodiment n is an integer of 2 or 4. One embodiment of the invention is a conjugate characterized by comprising an antifusogenic peptide at each C-terminus of the heavy chains or at each N-terminus of the light chains of the anti-CD4 antibody. In this embodiment two antifusogenic peptides are conjugated to one anti-CD4 antibody. In another embodiment the conjugate is characterized by comprising an antifusogenic peptide at each C-terminus of the heavy chains and at each N-terminus of the light chains. In this embodiment four antifusogenic peptides are conjugated to one anti-CD4 antibody.

The antifusogenic peptide which is introduced at a terminus of a mAb CD4 heavy and/or light chain(s) is small of size compared to the mAb CD4. For example, the smallest immunoglobulins, immunoglobulins of class G, have a molecular weight of approximately 150 kDa; an antifusogenic peptide has in one embodiment a size (molecular weight) of less than 12.5 kDa, which is equivalent to about 100 amino acids, in general less than 7.5 kDa, which is equivalent to about 60 amino acids. The antifusogenic peptide has in one embodiment an amino acid sequence of from 5 to 100 amino acid residues, preferably of from 10 to 75 amino acid residues, more preferably of from 15 to 50 amino acid residues. The conjugates of the current invention are useful for pharmaceutic, therapeutic, and/or diagnostic applications. The number of antifusogenic peptides, which can be conjugated to mAb CD4 heavy and/or light chain(s), is from one to the combined number of amino- and carboxy-termini of the anti-CD4 antibody's polypeptide chains. As the current invention encompasses different forms of anti-CD4 antibodies the possible number of antifusogenic peptides can vary. In case of an anti-CD4 antibody comprising two heavy and two light chains the combined number of amino-termini (N-termini) and carboxy-termini (C-termini) is eight, which is at the same time the totaling maximum number of conjugated antifusogenic peptides possible; in case e.g. of an anti-CD4 antibody fragment such as a single chain antibody (scFv) the combined number of termini and therefore the maximum number of conjugatable antifusogenic peptides is two. If a single antifusogenic peptide is conjugated to mAb CD4, the peptide can occupy any one of the termini of the anti-CD4 antibody chains. Likewise, if the maximum possible number of peptides is conjugated to mAb CD4, all termini are occupied by a single peptide. If the number of peptides, which are conjugated to mAb CD4, is smaller than the maximum possible number, different distributions of the peptides at the termini of the anti-CD4 antibody chains are possible. For example, if four peptides are conjugated to an immunoglobulin of the G or E class, five different combinations are possible (see Table 1). In two combinations all termini of one kind, i.e. all four amino-termini or all four carboxy-termini of the anti-CD4 antibody chains, are each conjugated to one single antifusogenic peptide. The other termini are not conjugated. This results in one embodiment in an allocation of the modifications/conjugations in one area of the anti-CD4 antibody. In the other cases the polypeptides are conjugated to a number of both termini. Within these combinations the conjugated peptides are allocated to different areas of the anti-CD4 antibody. In either case the sum of conjugated termini is four.

**Table 1: Possible combination for the conjugation of four peptides to the termini of an anti-CD4 antibody composed of four polypeptide chains.**

| **number of occupied amino-termini** | **number of occupied carboxy-termini** | **total number of occupied termini** |
|---|---|---|
| 4 | 0 | 4 |
| 3 | 1 | 4 |
| 2 | 2 | 4 |
| 1 | 3 | 4 |
| 0 | 4 | 4 |

The amino acid sequences of the conjugated antifusogenic peptides can be different, similar, or identical. In one embodiment the amino acid sequence identity is in the range of from 90% to less than 100%; these amino acid sequences and the corresponding peptides are defined as similar. In a preferred embodiment the antifusogenic peptides are identical, i.e. have an amino acid identity of 100% and the same length.

In one embodiment the conjugate according to the invention is characterized i) in comprising two light chain variable domains of SEQ ID NO: 02, two chain conjugates of type (2) each comprising a heavy chain variable domain of SEQ ID NO: 08, a linker of SEQ ID NO: 75 or 76, and an antifusogenic peptide of SEQ ID NO: 42 or 83, ii) in comprising two light chain variable domains of SEQ ID NO: 04, two chain conjugates of type (2) each comprising a heavy chain variable domain of SEQ ID NO: 10, a linker of SEQ ID NO: 75 or 76, and an antifusogenic peptide of SEQ ID NO: 42 or 83, iii) in comprising two light chain variable domains of SEQ ID NO: 05, two chain conjugates of type (2) each comprising a heavy chain variable domain of SEQ ID NO: 11, a linker of SEQ ID NO: 75 or 76, and an antifusogenic peptide of SEQ ID NO: 42 or 83, or iv) in comprising two light chain variable domains of SEQ ID NO: 06, two chain conjugates of type (2) each comprising a heavy chain variable domain of SEQ ID NO: 12, a linker of SEQ ID NO: 75 or 76, and an antifusogenic peptide of SEQ ID NO: 42 or 83.

The conjugation between the antifusogenic peptide and the anti-CD4 antibody is performed on the nucleic acid level. Therefore a peptide bond is formed between the antifusogenic peptide and the anti-CD4 antibody chain with or without an intermediate linker. Thus, either the carboxy-terminal amino acid of the antifusogenic peptide is conjugated to the amino-terminal amino acid of an anti-CD4 antibody chain, with or without an intermediate linker, or a carboxy-terminal amino acid of the anti-CD4 antibody chain is conjugated to the amino-terminal amino acid of the antifusogenic peptide, with or without an intermediate linker, or both termini of the anti-CD4 antibody chain are conjugated to an antifusogenic peptide each with or without an intermediate linker. For the recombinant production of the antifusogenic peptide-anti-CD4 antibody-conjugate according to the invention one or more nucleic acid molecules encoding different polypeptides are required, in one embodiment two to four nucleic acid molecules are employed. In one embodiment two nucleic acid molecules are employed. These nucleic acid molecules encode the different anti-CD4 antibody polypeptide chains of the conjugate and are in the following referred to as structural genes. They can be located on the same expression plasmid (vector) or can alternatively be located on different expression plasmids (vectors). The assembly of the conjugate preferably takes place before the secretion of the conjugate and, thus, within the expressing cell. Therefore, the nucleic acid molecules encoding the polypeptide chains of the conjugate are in one embodiment expressed in the same host cell. If after recombinant expression a mixture of conjugates is obtained, the conjugates can be separated and purified by methods known to a person skilled in the art. These methods are well established and widespread used for immunoglobulin purification and are employed either alone or in combination. Such methods are, for example, affinity chromatography using microbial-derived proteins (e.g. protein A or protein G affinity chromatography), ion exchange chromatography (e.g. cation exchange (carboxymethyl resins), anion exchange (amino ethyl resins) and mixed-mode exchange chromatography), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)-and Cu(II)-affinity material), size exclusion chromatography, and preparative electrophoretic methods (such as gel electrophoresis, capillary electrophoresis) (Vijayalakshmi, M.A., Appl. Biochem. Biotech. 75 (1998) 93-102). With recombinant engineering methods known to a person skilled in the art the conjugates can be tailor-made on the nucleic acid/gene level. The nucleic acid sequences encoding immunoglobulin light and heavy chains are known and can be obtained for example from genomic databases. Likewise the nucleic acid sequences encoding antifusogenic peptides are known or can easily be deduced from their corresponding amino acid sequence. The elements required for the construction of an expression plasmid for the expression of the conjugate according to the invention are, for example, an expression cassette for the anti-CD4 antibody light chain in its natural and/or conjugated form, an expression cassette for the anti-CD4 antibody heavy chain in its natural and/or conjugated form (alternatively the anti-CD4 antibody light chain and the anti-CD4 antibody heavy chain structural gene can be contained in the same expression cassette, e.g. as bicistronic expression element), a selection marker, and an E.coli replication as well as selection unit. Expression cassettes comprise a promoter, a DNA segment encoding a secretion signal sequence, the structural gene, and a terminator/polyadenylation signal. The elements are assembled in an operatively linked form either on one plasmid encoding all chains of the conjugate, or on two or more plasmids each encoding one or more chains of the conjugate. For the expression of the structural genes the plasmid(s) is (are) introduced into a suitable host cell. Proteins are produced in mammalian cells such as CHO cells, NS0 cells, Sp2/0 cells, COS cells, HEK cells, K562 cells, BHK cells, PER.C6^{®} cells, and the like. In one embodiment the conjugate is expressed in a CHO cell, or a BHK cell, or a HEK cell. The regulatory elements of the plasmid have to be selected in a way that they are functional in the selected host cell. For expression the host cell is cultivated under conditions suitable for the expression of the conjugate. The expressed conjugate chains are functionally assembled. The fully processed antifusogenic peptide-anti-CD4 antibody-conjugate is secreted into the medium.

An "expression plasmid" is a nucleic acid providing all required elements for the expression of the comprised structural gene(s) in a host cell. Typically, an expression plasmid comprises a prokaryotic plasmid propagation unit, e.g. for E. coli, comprising an origin of replication, and a selectable marker, an eukaryotic selection marker, and one or more expression cassettes for the expression of the structural gene(s) of interest each comprising a promoter, a structural gene, and a transcription terminator including a polyadenylation signal. Gene expression is usually placed under the control of a promoter, and such a structural gene is said to be "operably linked to" the promoter. Similarly, a regulatory element and a core promoter are operably linked if the regulatory element modulates the activity of the core promoter.

One aspect of the current invention is thus a method for the production of a conjugate according to the invention, comprising the following steps:
a) cultivating a cell containing nucleic acid molecules encoding a conjugate according to the invention under conditions suitable for the expression of the conjugate,
b) recovering the conjugate from the cell or the supernatant.

The term "under conditions suitable for the expression of the conjugate" denotes conditions which are used for the cultivation of a cell expressing a polypeptide and which are known to or can easily be determined by a person skilled in the art. It is known to a person skilled in the art that these conditions may vary depending on the type of cell cultivated and type of polypeptide expressed. In general the cell is cultivated at a temperature, e.g. between 20 °C and 40 °C, and for a period of time sufficient to allow effective production of the conjugate, e.g. for of from 4 to 28 days, in a volume of 0.01 to 10⁷ liter.

As used herein, "pharmaceutically acceptable carrier" includes any and all solvents, dispersion media, coatings, antibacterial and antifungal agents, isotonic and absorption/resorption delaying agents, and the like that are physiologically compatible. Preferably, the carrier is suitable for injection or infusion. Pharmaceutically acceptable carriers include sterile aqueous solutions or dispersions and sterile powders for the preparation of sterile injectable solutions or dispersion. The use of such media and agents for pharmaceutically active substances is known in the art. In addition to water, the carrier can be, for example, an isotonic buffered saline solution.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skilled in the art.

Actual dosage levels of the active ingredients in the pharmaceutical compositions of the present invention may be varied so as to obtain an amount of the active ingredient, which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, other drugs, compounds and/or materials used in combination with the particular compositions employed, the age, sex, weight, condition, general health and prior medical history of the patient being treated, and like factors well known in the medical arts.

The invention preferably comprises the use of a conjugate according to the invention for the treatment of a patient suffering from immunodeficiency syndromes such as AIDS.

The following examples, sequence listing, and figures are provided to aid the understanding of the present invention, the true scope of which is set forth in the appended claims. It is understood that modifications can be made in the procedures set forth without departing from the spirit of the invention.

### Description of the Figures

- Figure 1:: Plasmid map of mAb CD4 κ-light chain expression vector 6310.
- Figure 2:: Plasmid map of mAb CD4 γl -heavy chain expression vector 6309.
- Figure 3:: Plasmid map of mAb CD4 γl-heavy chain conjugate expression vector 6303.
- Figure 4:: Plasmid map of mAb CD4 γl-heavy chain conjugate expression vector 6304.

### Antibody nomenclature

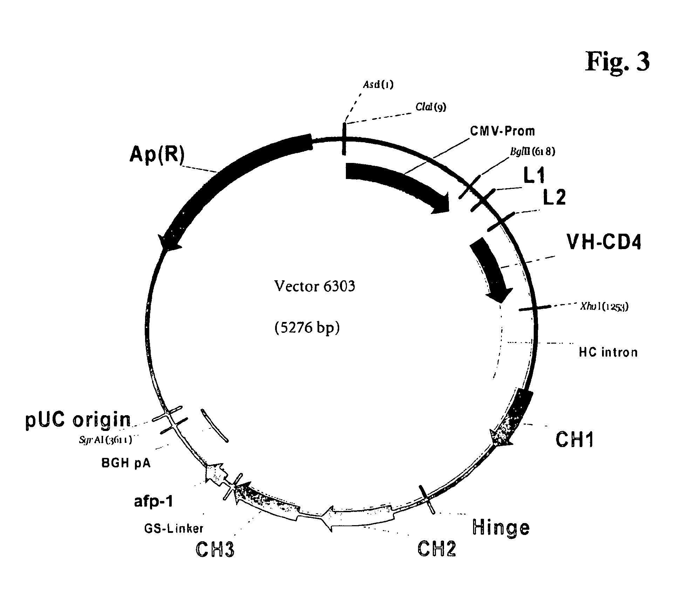

Variable domain 1 is reported in SEQ ID NO: 10, 15, 45, and 56 of US 5,871,732.
Variable 2 and 4 are reported in Reimann, K.A., et al., Aids Res. Human Retrovir. 13 (1997) 933-943.
Variable domain 3 is reported in Figures 3, 4, 12, and 13 of WO 91/009966.

### Anti-CD4 antibody sequences, sequences of antifusogenic peptides and sequences of peptidic linkers

SEQ ID NO: 01 murine light chain variable domain 1
SEQ ID NO: 02 chimeric light chain variable domain 1
SEQ ID NO: 03 chimeric light chain 1
SEQ ID NO: 04 chimeric light chain variable domain 2
SEQ ID NO: 05 chimeric light chain variable domain 3
SEQ ID NO: 06 chimeric light chain variable domain 4
SEQ ID NO: 07 murine heavy chain variable domain 1
SEQ ID NO: 08 chimeric heavy chain variable domain 1
SEQ ID NO: 09 chimeric heavy chain 1
SEQ ID NO: 10 chimeric heavy chain variable domain 2
SEQ ID NO: 11 chimeric heavy chain variable domain 3
SEQ ID NO: 12 chimeric heavy chain variable domain 4
SEQ ID NO: 13 light chain CDR1
SEQ ID NO: 14 light chain CDR1
SEQ ID NO: 15 light chain CDR1
SEQ ID NO: 16 light chain CDR1
SEQ ID NO: 17 light chain CDR2
SEQ ID NO: 18 light chain CDR2
SEQ ID NO: 19 light chain CDR2
SEQ ID NO: 20 light chain CDR3
SEQ ID NO: 21 light chain CDR3
SEQ ID NO: 22 light chain CDR3
SEQ ID NO: 23 heavy chain CDR1
SEQ ID NO: 24 heavy chain CDR1
SEQ ID NO: 25 heavy chain CDR1
SEQ ID NO: 26 heavy chain CDR2
SEQ ID NO: 27 heavy chain CDR2
SEQ ID NO: 28 heavy chain CDR2
SEQ ID NO: 29 heavy chain CDR3
SEQ ID NO: 30 heavy chain CDR3
SEQ ID NO: 31 heavy chain CDR3
SEQ ID NO: 32 human γ1 heavy chain constant region
SEQ ID NO: 33 human γ4 heavy chain constant region
SEQ ID NO: 34 human κ light chain constant domain
SEQ ID NO: 35 C34
SEQ ID NO: 36 T20
SEQ ID NO: 37 T1249
SEQ ID NO: 38 T651
SEQ ID NO: 39 T2635
SEQ ID NO: 40 N36
SEQ ID NO: 41 DP107
SEQ ID NO: 42 afp-1
SEQ ID NO: 43 HIV-1 gp41 ectodomain variant single mutant: I568P
SEQ ID NO: 44 HIV-1 gp41 ectodomain variant quadruple mutant: I568P, L550E, L566E, I580E
SEQ ID NO: 45 HIV-1 gp41
SEQ ID NO: 46-76 linker peptides 80,81
SEQ ID NO: 77 Amino acid sequence of mature mAb CD4 κ-light chain
SEQ ID NO: 78 Amino acid sequence of mature mAb CD4 γ1-heavy chain with afp-1
SEQ ID NO: 79 Amino acid sequence of mature mAb CD4 conjugate heavy chain
SEQ ID NO: 82 Amino acid sequence of mature mAb CD4 γ1-heavy chain with T-651
SEQ ID NO: 83 afp-2

**Table 2: Linker**

| **No.** | **Linker peptides** | **SEQ ID NO:** |
|---|---|---|
| 1 | LSLSPGK | 46 |
| 2 | LSPNRGEC | 47 |
| 3 | [GQ₄]₃ | 48 |
| 4 | [GQ₄]₃G | 49 |
| 5 | [GQ₄]₃GNN | 50 |
| 6 | GGG[SG₄]₂SGG | 51 |
| 7 | GGG[SG₄]₂SGN | 52 |
| 8 | [SG₄]₃ | 53 |
| 9 | [SG₄]₃G | 54 |
| 10 | G[SG₄]₃T | 55 |
| 11 | [SG₄]₃GG | 56 |
| 12 | [SG₄]₃GGT | 57 |
| 13 | [SG₄]₃GGN | 58 |
| 14 | [SG₄]₃GAS | 59 |
| 15 | [SG₄]₅ | 60 |
| 16 | [SG₄]₅G | 61 |
| 17 | [SG₄]₅GG | 62 |
| 18 | [SG₄]₅GAS | 63 |
| 19 | G(S)₁₅G | 64 |
| 20 | G(S)₁₅GAS | 65 |
| 21 | G | - |
| 22 | N | - |
| 23 | GST | - |
| 24 | [(G)₄S]₃GAS | 66 |
| 25 | [(G)₄S]₃G | 67 |
| 26 | [(G)₄S]₅G | 68 |
| 27 | [(G)₄S]₃GG | 69 |
| 28 | [(G)₄S]₅GG | 70 |
| 29 | LSLSGG | 71 |
| 30 | LSLSPGG | 72 |
| 31 | [G₃S]₅ | 73 |
| 32 | [G₃S]₅GGG | 74 |
| 33 | G₃[SG₄]₂SG | 75 |
| 34 | G₃[SG₄]₂SG₂ | 76 |
| 35 | [G₄S]₃GGN | 80 |
| 36 | K[G₄S]₃GGN | 81 |

### Examples

### Materials & Methods

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991). Amino acids of antibody chains are numbered and referred to according to EU numbering (Edelman, G.M., et al., Proc. Natl. Acad. Sci. USA 63 (1969) 78-85; Kabat, E.A., et al., Sequences of Proteins of Immunological Interest, 5th ed., Public Health Service, National Institutes of Health, Bethesda, MD, (1991)).

### Recombinant DNA techniques

Standard methods were used to manipulate DNA as described in Sambrook, J. et al., Molecular cloning: A laboratory manual; Cold Spring Harbor Laboratory Press, Cold Spring Harbor, New York, 1989. The molecular biological reagents were used according to the manufacturer's instructions.

### Gene synthesis

Desired gene segments were prepared from oligonucleotides made by chemical synthesis. The 100 - 600 bp long gene segments, which are flanked by singular restriction endonuclease cleavage sites, were assembled by annealing and ligation of oligonucleotides including PCR amplification and subsequently cloned into the pCR2.1-TOPO-TA cloning vector (Invitrogen Corp., USA) via A-overhangs. The DNA sequence of the subcloned gene fragments were confirmed by DNA sequencing.

### Protein determination

The protein concentration of the conjugate was determined by determining the optical density (OD) at 280 nm, using the molar extinction coefficient calculated on the basis of the amino acid sequence.

### Example1

### Making of the expression plasmids

The gene segments encoding an anti-CD4 antibody light chain variable domain (V_{L}) and the human kappa-light chain constant domain (C_{L}) were joined as were gene segments for the anti-CD4 antibody heavy chain variable domain (V_{H}) and the human γ1-heavy chain constant domains (C_{H}1-Hinge-C_{H}2-C_{H}3).

In the case of mAb CD4 of SEQ ID NO: 77/78 the heavy and light chain variable domains are derived from a mouse antibody which were humanized as described e.g. by Reimann, K.A., et al., Aids Res. Human Retrovir. 13 (1997) 933-943 or in US 5,871,732, and the heavy and light chain constant domains are derived from a human antibody (C-kappa and IgG1).

Subsequently, the gene segment encoding a complete anti-CD4 antibody light chain was joined at the N- and/or C-terminus with a nucleic acid encoding an antifusogenic peptide including a connecting linker sequence and/or the gene segment encoding a complete anti-CD4 antibody heavy chain was joined at the N-and/or C-terminus with a nucleic acid encoding an antifusogenic peptide including a connecting linker sequence.

### a) Vector 6310

Vector 6310 is an expression plasmid e.g. for transient expression of a mAb CD4 light chain (genomically organized expression cassette; exon-intron organization) in HEK293 cells.

Beside the mAb CD4 κ-light chain expression cassette this vector contains:
- a neomycin resistance gene as a selectable marker,
- an origin of replication from the vector pUC18 which allows replication of this plasmid in E. coli, and
- a ß-lactamase gene which confers ampicillin resistance in E. coli.

The transcription unit of the mAb CD4 κ-light chain gene is composed of the following elements:
- the immediate early enhancer and promoter from the human cytomegalovirus,
- a 5'-untranslated region of a human antibody germline gene,
- a murine immunoglobulin heavy chain signal sequence including a signal sequence intron (signal sequence 1, intron, signal sequence 2 [L1-intron-L2]),
- the humanized anti-CD4 antibody mature variable κ-light chain encoding segment arranged with a splice donor site and a unique BamHI restriction site at the 3'-end,
- a truncated human κ-light chain intron 2,
- the human κ-light gene constant domain,
- the bovine growth hormone (bGH) polyadenylation ("poly A") signal sequence, and
- the unique restriction sites AscI and SgrAI at the 3'-end.

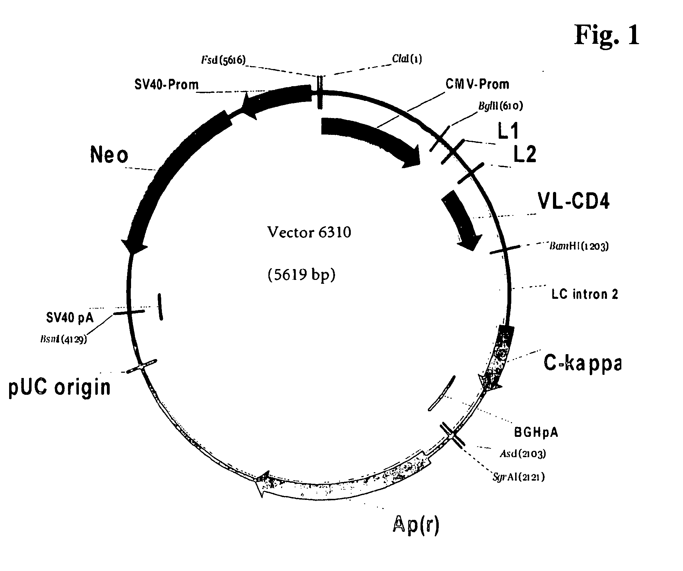

The plasmid map of the mAb CD4 κ-light chain expression vector 6310 is shown in Figure 1. The amino acid sequence of the mature (without signal sequence) mAb CD4 κ-light chain is shown in SEQ ID NO: 77.

### b) Vector 6309

Vector 6309 is an expression plasmid e.g. for transient expression of a mAb CD4 y1-heavy chain (genomically organized expression cassette; exon-intron organization) in HEK293 cells.

Beside the mAb CD4 γ1-heavy chain expression cassette this vector contains:
- an origin of replication from the vector pUC18 which allows replication of this plasmid in E. coli, and
- a beta-lactamase gene which confers ampicillin resistance in E. coli.

The transcription unit of the mAb CD4 γ1-heavy chain is composed of the following elements:
- the immediate early enhancer and promoter from the human cytomegalovirus,
- a 5'-untranslated region of a human antibody germline gene,
- a murine immunoglobulin heavy chain signal sequence including a signal sequence intron (signal sequence 1, intron, signal sequence 2 [L1-intron-L2]),
- the humanized anti-CD4 antibody mature variable heavy chain encoding segment arranged with a splice donor site and a unique Xhol restriction site at the 3'-end,
- a mouse/human heavy chain hybrid intron 2
- the genomic human γ1-heavy gene constant domains containing the L234A and L235A mutation,
- the bovine growth hormone (bGH) polyadenylation ("poly A") signal sequence, and
- the unique restriction site SgrAI at the 3'-end.

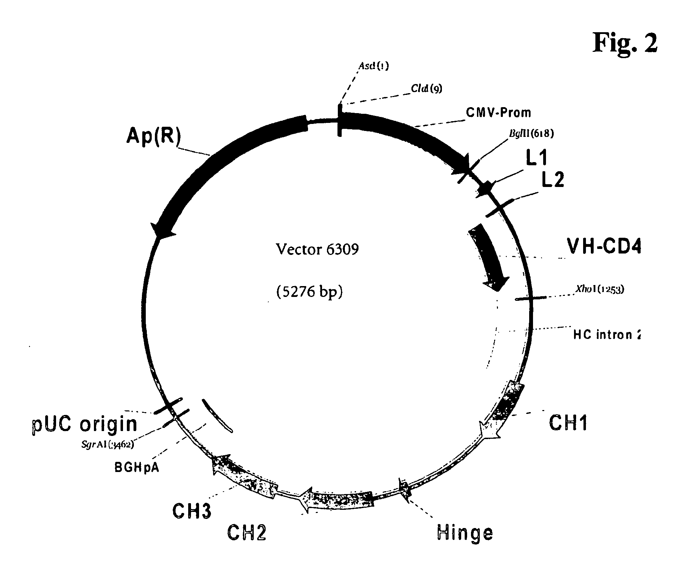

The plasmid map of the mAb CD4 γ1-heavy chain expression vector 6309 is shown in Figure 2. The amino acid sequence of the mature (without signal sequence) mAb CD4 γ1-heavy chain is shown in SEQ ID NO: 78.

### c) Vector 6303

Vector 6303 is an expression plasmid e.g. for transient expression of a chimeric peptide-anti-CD4 antibody γ1-heavy chain conjugate (genomically organized expression cassette; exon-intron organization) in HEK293 cells.

The vector 6303 is derived from vector 6309 in that way that the mAb CD4 γ1-heavy chain is joint at the last but one C-terminal amino acid, i.e. the C-terminal lysine residue of the heavy chain is removed, with a nucleic acid encoding the antifusogenic peptide afp-1 (SEQ ID NO: 42) and the peptidic glycine-serine linker of SEQ ID NO: 75.

Beside the chimeric peptide anti-CD4 antibody γ1-heavy chain conjugate expression cassette this vector contains:
- an origin of replication from the vector pUC18 which allows replication of this plasmid in E. coli, and
- a beta(β)-lactamase gene which confers ampicillin resistance in E. coli.

The transcription unit of the chimeric peptide-anti-CD4 antibody γ1-heavy chain conjugate is composed of the following elements:
- the immediate early enhancer and promoter from the human cytomegalovirus,
- a 5'-untranslated region of a human antibody germline gene,
- a murine immunoglobulin heavy chain signal sequence including a signal sequence intron (signal sequence 1, intron, signal sequence 2 [L1-intron-L2]),
- the humanized anti-CD4 antibody mature variable heavy chain encoding segment arranged with a splice donor site and a unique XhoI restriction site at the 3'-end,
- a mouse/human heavy chain hybrid intron containing the L234A and L235A mutations,
- the peptidic serine-glycine linker sequence of SEQ ID NO: 75,
- the antifusogenic peptide afp-1 of SEQ ID NO: 42,
- the bovine growth hormone (bGH) polyadenylation ("poly A") signal sequence, and
- the unique restriction site SgrAI at the 3'-end.

The plasmid map of the mAb CD4 γ1-heavy chain conjugate expression vector 6303 is shown in Figure 3. The amino acid sequence of the mature (without signal sequence) conjugate heavy chain is shown in SEQ ID NO: 79.

### d) Vector 6304

Vector 6304 is an expression plasmid e.g. for transient expression of a chimeric peptide-anti-CD4 antibody γ1-heavy chain conjugate (genomically organized expression cassette; exon-intron organization) in HEK293 cells.

Beside the chimeric peptide anti-CD4 antibody γ1-heavy chain conjugate expression cassette this vector contains:
- an origin of replication from the vector pUC18 which allows replication of this plasmid in E. coli, and
- a beta(β)-lactamase gene which confers ampicillin resistance in E. coli.

The transcription unit of the chimeric peptide-anti-CD4 antibody γ1-heavy chain conjugate is composed of the following elements:
- the immediate early enhancer and promoter from the human cytomegalovirus,
- a 5'-untranslated region of a human antibody germline gene,
- a murine immunoglobulin heavy chain signal sequence including a signal sequence intron (signal sequence 1, intron, signal sequence 2 [L1-intron-L2]),
- the humanized anti-CD4 antibody mature variable heavy chain encoding segment arranged with a splice donor site and a unique XhoI restriction site at the 3'-end,
- a mouse/human heavy chain hybrid intron containing the L234A and L235A mutations,
- the peptidic serine-glycine linker sequence of SEQ ID NO: 80,
- the antifusogenic peptide T-651 of SEQ ID NO: 38,
- the bovine growth hormone (bGH) polyadenylation ("poly A") signal sequence, and
- the unique restriction site SgrAI at the 3'-end.

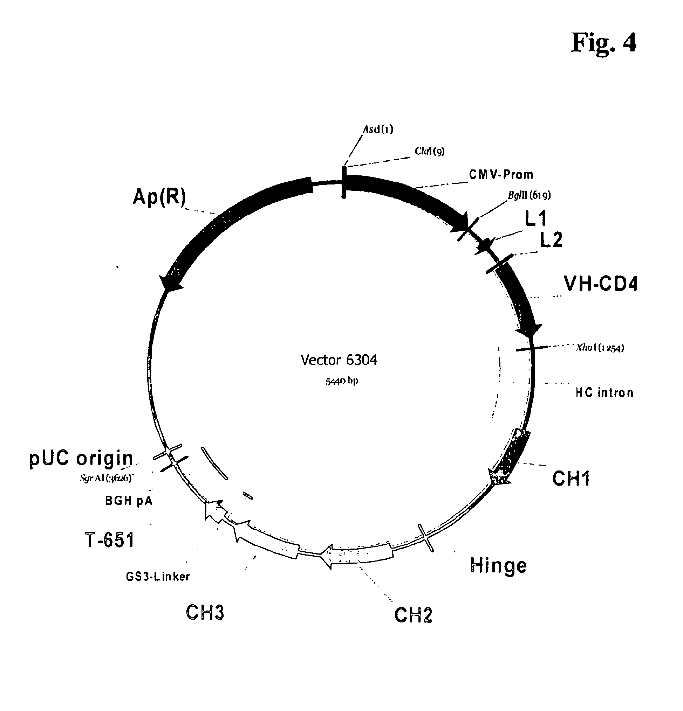

The plasmid map of the mAb CD4 γ1-heavy chain conjugate expression vector 6304 is shown in Figure 4. The amino acid sequence of the mature (without signal sequence) conjugate heavy chain is shown in SEQ ID NO: 82.

The vector 6304 is derived from vector 6309 in that way that the mAb CD4 γ1-heavy chain is joint at the last but one C-terminal amino acid, i.e. the C-terminal lysine residue of the heavy chain is removed, with a nucleic acid encoding the antifusogenic peptide T-651 (SEQ ID NO: 38) and the peptidic glycine-serine linker of SEQ ID NO: 80.

### e) Vector 6305

Vector 6305 is an expression plasmid e.g. for transient expression of a chimeric peptide-anti-CD4 antibody γ1-heavy chain conjugate (genomically organized expression cassette; exon-intron organization) in HEK293 cells.

Beside the chimeric peptide anti-CD4 antibody γ1-heavy chain conjugate expression cassette this vector contains:
- an origin of replication from the vector pUC18 which allows replication of this plasmid in E. coli, and
- a beta(β)-lactamase gene which confers ampicillin resistance in E. coli.

The transcription unit of the chimeric peptide-anti-CD4 antibody γ1-heavy chain conjugate is composed of the following elements:
- the immediate early enhancer and promoter from the human cytomegalovirus,
- a 5'-untranslated region of a human antibody germline gene,
- a murine immunoglobulin heavy chain signal sequence including a signal sequence intron (signal sequence 1, intron, signal sequence 2 [L1-intron-L2]),
- the humanized anti-CD4 antibody mature variable heavy chain encoding segment arranged with a splice donor site and a unique XhoI restriction site at the 3'-end,
- a mouse/human heavy chain hybrid intron containing the L234A and L235A mutations,
- the peptidic serine-glycine linker sequence of SEQ ID NO: 76,
- the antifusogenic peptide afp-2 of SEQ ID NO: 83,
- the bovine growth hormone (bGH) polyadenylation ("poly A") signal sequence, and
- the unique restriction site SgrAI at the 3'-end.

The vector 6305 is derived from vector 6309 in that way that the mAb CD4 γ1-heavy chain is joint at the last but one C-terminal amino acid, i.e. the C-terminal lysine residue of the heavy chain is removed, with a nucleic acid encoding the antifusogenic peptide afp-2 (SEQ ID NO: 83) and the peptidic glycine-serine linker of SEQ ID NO: 76.

### Example 2

### Making of the final expression plasmids

The fusion genes (heavy and/or light chain antibody fusion genes) comprising a mAb CD4 gene segment, an optional linker gene segment and an antifusogenic peptide gene segment have been assembled with known recombinant methods and techniques by connection of the according nucleic acid segments. The nucleic acid sequences encoding the peptidic linkers and antifusogenic polypeptides were each synthesized by chemical synthesis and then ligated into an E.coli plasmid for amplification. The subcloned nucleic acid sequences were verified by DNA sequencing.

### Example 3

### Transient expression of immunoglobulins and immunoglobulin variants in HEK293 EBNA cells

Recombinant anti-CD4 antibodies and anti-CD4 antibody-variants were generated by transient transfection of adherent growing HEK293-EBNA cells (human embryonic kidney cell line 293 expressing Epstein-Barr-Virus nuclear antigen; American type culture collection deposit number ATCC # CRL-10852) cultivated in DMEM (Dulbecco's modified Eagle's medium, Gibco) supplemented with 10 % ultra-low IgG FCS (fetal calf serum, Gibco), 2 mM Glutamine (Gibco), 1 % volume by volume (v/v) nonessential amino acids (Gibco) and 250 µg/ml G418 (Roche Molecular Biochemicals). For transfection FUGENE™ 6 Transfection Reagent (Roche Molecular Biochemicals) was used in a ratio of reagent (µl) to DNA (µg) ranging from 3:1 to 6:1. Light and heavy chains including antifusogenic peptide-anti-CD4 antibody conjugate light and heavy chains were expressed from two different plasmids using a molar ratio of light chain to heavy chain encoding plasmid ranging from 1:2 to 2:1, respectively. Antifusogenic peptide-anti-CD4 antibody conjugates containing cell culture supernatants were harvested at day 4 to 11 after transfection. General information regarding the recombinant expression of human immunoglobulins in e.g. HEK293 cells is given in: Meissner, P. et al., Biotechnol. Bioeng. 75 (2001) 197-203.

### Example 4

### Expression analysis using SDS PAGE, Western blotting transfer and detection with immunoglobulin specific antibody conjugates

The expressed and secreted antifusogenic peptide-anti-CD4 antibody conjugates were processed by sodium dodecyl sulfate (SDS) polyacrylamide gel electrophoresis (SDS-PAGE), and the separated anti-CD4-antibody and antifusogenic peptide-anti-CD4-antibody-conjugate chains were transferred to a membrane from the gel and subsequently detected by an immunological method.

### SDS-PAGE

LDS sample buffer, fourfold concentrate (4x): 4 g glycerol, 0.682 g TRIS-Base, 0.666 g TRIS-hydrochloride, 0.8 g LDS (lithium dodecyl sulfate), 0.006 g EDTA (ethylene diamin tetra acid), 0.75 ml of a 1 % by weight (w/w) solution of Serva Blue G250 in water, 0.75 ml of a 1 % by weight (w/w) solution of phenol red, add water to make a total volume of 10 ml.

The culture broth containing the secreted antifusogenic peptide-anti-CD4 antibody conjugate was centrifuged to remove cells and cell debris. An aliquot of the clarified supernatant was admixed with 1/4 volumes (v/v) of 4xLDS sample buffer and 1/10 volume (v/v) of 0.5 M 1,4-dithiotreitol (DTT). Then the samples were incubated for 10 min. at 70°C and protein separated by SDS-PAGE. The NuPAGE^{®} Pre-Cast gel system (Invitrogen) was used according to the manufacturer's instruction. In particular, 10 % NuPAGE^{®} Novex^{®} Bis-TRIS Pre-Cast gels (pH 6.4) and a NuPAGE^{®} MOPS running buffer was used.

### Western blot

Transfer buffer: 39 mM glycine, 48 mM TRIS-hydrochloride, 0.04 % by weight (w/w) SDS, and 20 % by volume methanol (v/v).

After SDS-PAGE the separated antifusogenic peptide-anti-CD4 antibody conjugate chains were transferred electrophoretically to a nitrocellulose filter membrane (pore size: 0.45 µm) according to the "Semidry-Blotting-Method" of Burnette (Burnette, W.N., Anal. Biochem. 112 (1981) 195-203).

### Immunological detection

TBS-buffer: 50 mM TRIS-hydrochloride, 150 mM NaCl, adjusted to pH 7.5 Blocking solution: 1 % (w/v) Western Blocking Reagent (Roche Molecular Biochemicals) in TBS-buffer

### TBST-Buffer: 1xTBS-buffer with 0.05 % by volume (v/v) Tween-20

For immunological detection the western blotting membranes were incubated with shaking at room temperature two times for 5 minutes in TBS-buffer and once for 90 minutes in blocking solution.

### Detection of the peptide immunoglobulin conjugate chains

Heavy chain: For detection of the heavy chain of the antifusogenic peptide-anti-CD4 antibody conjugate a purified rabbit anti-human IgG antibody conjugated to a peroxidase was used (DAKO, Code No. P 0214).

Light chain: The light chain of the antifusogenic peptide-anti-CD4 antibody conjugate was detected with a purified peroxidase conjugated rabbit anti-human kappa light chain antibody (DAKO, Code No. P 0129).

For visualization of the antibody light and heavy chains washed and blocked Western blot membranes were first incubated in case of a heavy chain with a purified rabbit anti-human IgG antibody conjugated to a peroxidase or in case of a light chain with a purified peroxidase conjugated rabbit anti-human kappa light chain antibody in a 1:10,000 dilution in 10 ml blocking solution at 4°C with shaking over night. After washing the membranes three times with TBTS-buffer and once with TBS buffer for 10 min. at room temperature, the Western-blot membranes were developed with a Luminol/peroxid-solution generating chemiluminescence (Lumi-Light^{PLUS} Western Blotting Substrate, Roche Molecular Biochemicals). Therefore the membranes were incubated in 10 ml Luminol/peroxid-solution for 10 seconds to 5 minutes and the emitted light was detected afterwards with a LUMI-Imager F1 Analysator (Roche Molecular Biochemicals) and/or was recorded with an x-ray-film. The intensity of the spots was quantified with the LumiAnalyst Software (Version 3.1).

### Multiple-staining of immunoblots

The secondary peroxidase-labeled antibody conjugate used for the detection can be removed from the stained blot by incubating the membrane for one hour at 70°C in I M TRIS-hydrochloride-buffer (pH 6.7) containing 100 mM beta-mercaptoethanol and 20 % (w/v) SDS. After this treatment the blot can be stained with a different secondary antibody a second time. Prior to the second detection the blot is washed three times at room temperature with shaking in TBS-buffer for 10 minutes each.

### Example 5

### Affinity purification, dialysis and concentration of peptide immunoglobulin conjugates

The expressed and secreted antifusogenic peptide-anti-CD4 antibody conjugates were purified by affinity chromatography using Protein A-Sepharose™ CL-4B (GE Healthcare former Amersham Bioscience, Sweden) according to known methods. Briefly, after centrifugation (10,000 g for 10 minutes) and filtration through a 0.45 µm filter the peptide immunoglobulin conjugate containing clarified culture supernatants were applied on a Protein A-Sepharose™ CL-4B column equilibrated with PBS buffer (10 mM Na₂HPO₄, 1 mM KH₂PO₄, 137 mM NaCl and 2.7 mM KCI, pH 7.4). Unbound proteins were washed out with PBS equilibration buffer and 0.1 M citrate buffer, pH 5.5. The antifusogenic peptide-anti-CD4 antibody conjugates were eluted with 0.1 M citrate buffer, pH 3.0, and the conjugate containing fractions were neutralized with 1 M TRIS-Base. Then, the antifusogenic peptide-anti-CD4 antibody conjugates were extensively dialyzed against PBS buffer at 4 °C, concentrated with a Ultrafree^{®}-CL Centrifugal Filter Unit equipped with a Biomax-SK membrane (Millipore Corp., USA) and stored in an ice-water bath at 0 °C. The integrity of the conjugates was analyzed by SDS-PAGE in the presence and absence of a reducing agent and staining with Coomassie brilliant blue as described in Example 4. Aggregation of antifusogenic peptide-anti-CD4 antibody conjugates was analyzed by analytical size exclusion chromatography.

### Example 6

### Deglycosylation of peptide immunoglobulin conjugates

N-linked carbohydrates of anti-CD4 antibodies and antifusogenic peptide-anti-CD4 antibody conjugates were cleaved off by enzymatic treatment with Peptide-N-Glycosidase F (PNGaseF, Roche Molecular Biochemicals, Mannheim, Germany or Prozyme, San Leandro, CA). Therefore, the anti-CD4 antibodies and antifusogenic peptide-anti-CD4 antibody conjugates were incubated at 37°C for 12-24 h using 50 mU PNGaseF per mg N-glycosylated protein in PBS buffer at a protein concentration of about 2 mg/ml. Thereafter the Peptide-N-Glycosidase F was separated by preparative gel filtration according to known methods. Briefly, PNGaseF treated anti-CD4-antibodies and antifusogenic peptide-anti-CD4 antibody conjugates were applied on a Superose™ 1.2 10/300 GL column (GE Healthcare former Amersham Bioscience, Sweden) equilibrated with PBS buffer (10 mM Na₂HPO₄, 1 mM KH₂PO₄, 137 mM NaCl and 2.7 mM KCl, pH 7.4) and then eluted with equilibration buffer at a flow rate of 0.5-1.0 ml/min using the Äkta explorer chromatography system from Amersham Bioscience (GE Healthcare former Amersham Bioscience, Sweden).

### Example 7

### Single-cycle antiviral activity assay.

For the production of pseudotyped NL-Bal viruses, plasmid pNL4-3Δenv (HIV pNL4-3 genomic construct with a deletion within the env gene) and pCDNA3.1/NL-BAL *env* [pcDNA3.1 plasmid containing NL-Bal env gene (obtained from NIBSC Centralized Facility for AIDS Reagents)] were co-transfected into the HEK 293FT cell line (Invitrogen Corp.), cultured in Dulbecco's' modified minimum medium (DMEM) containing 10 % fetal calf serum (FCS), 100 U/mL Penicillin, 100 µg/mL Streptomycin, 2 mM L-glutamine and 0.5 mg/mL geniticin (all media from Invitrogen/Gibco). The supernatants containing pseudotyped viruses were harvested two days following transfection, and cellular debris was removed by filtration through a 0.45 µm pore size PES (polyethersulfone) filter (Nalgene) and stored at -80 °C in aliquots. For normalization in assay performance, virus stock aliquots were used to infect JC53-BL (US NIH Aids Reagent Program) cells yielding approximately 1.5 x 10⁵ RLU (relative light units) per well. Test antifusogenic peptide-anti-CD4 antibody conjugates, parent anti-CD4 antibody, reference antibodies and reference antifusogenic peptide (T-651) were serially diluted in 96-well plates. The assay was carried out in quadruplicates. Each plate contained cell control and virus control wells. The equivalent of 1.5 x 10⁵ RLU of virus stocks were added to each well, then 2.5 x 10⁴ JC53-BL cells were added to each well, with a final assay volume of 200 µl per well. After 3 day incubation at 37 °C, 90 % Relative Humidity, and 5 % CO₂, media were aspirated and 50 µl of Steady-Glo^{®} Luciferase Assay System (Promega) was added to each well. The assay plates were read on a Luminometer (Luminoskan, Thermo Electron Corporation) after 10 minutes of incubation at room temperature. Percent inhibition of luciferase activity was calculated for each dose point after subtracting the background, and IC₅₀ and IC₉₀-values were determined by using XLfit curve fitting software for Excel (version 3.0.5 Build12; Microsoft). Results are shown in Table 3.

**Table 3: Antiviral activity of antifusogenic polypeptides, antibodies and antifusogenic peptide-anti-CD4 antibody conjugates (by weight).**

| **Compound** | **Antiviral activity** | |
|---|---|---|
| | **NL-BAL (R5)** | **NL-4-3 (X4)** |
| | **IC₅₀ [ng/mL] / IC₉₀ [ng/mL]** | **IC₅₀ [ng/mL] / IC₉₀ [ng/mL]** |
| Reference antibody 1 (inert) | inactive / inactive | inactive / inactive |
| Reference antibody 2 (inert) | inactive / inactive | inactive / inactive |
| T-651 | 21.5/181.9 | 169.2 / 1099.7 |
| afp-1 | 19.6 / - | |
| mAb CD4 (6310/6309) | maximal inhibition = 50% | Maximal inhibition = 20% |
| antifusogenic peptide-anti CD4 | 6.1 / 32.2 | 43.9 / 123.9 |
| antibody conjugate (6310/6303) | | |

**Table 4: Antiviral activity of antifusogenic polypeptides, antibodies and antifusogenic peptide-anti-CD4 antibody conjugates (by molarity).**

| **Compound** | **Antiviral activity** | |
|---|---|---|
| | **NL-BAL (R5)** | **NL-4-3 (X4)** |
| | **IC₅₀ [nM] / IC₉₀ [nM] IC₅₀ [nM] / IC₉₀ [nM]** | |
| Reference antibody 1 (inert) | inactive / inactive | Inactive / inactive |
| Reference antibody 2 (inert) | inactive / inactive | Inactive / inactive |
| T-651 | 5.4 / 45 | 42 / 275 |
| mAb CD4 (6310/6309) | maximal inhibition = 50% | Maximal inhibition = 20% |
| antifusogenic peptide-anti CD4 | 0.038 / 0.201 | 0.275 / 0.774 |
| antibody conjugate (6310/6303) | | |

### Example 8

### Cell-Cell Fusion Assay

At day 1, gp160-expressing HeLa cells (2 x 10⁴ cells / 50 µl / well) are seeded in a white 96 microtiter plate in DMEM medium supplemented with 10 % FCS and 2 µg/ml doxycycline. At day 2, 100 µl of supernatant sample or antibody control per well is added in a clear 96 microtiter plate. Then 100 µl containing 8x10⁴ CEM-NKr-Luc suspension cells in medium are added and incubated 30 min. at 37 °C. The HeLa cell culture medium is aspirated from the 96 well plate, 100 µl from the 200 µl antibody/CEM-NKr-Luc mixture is added and incubated over night at 37 °C. At day 3, 100 µl/well Bright-Glo™ Luciferase assay substrate (1,4-dithiothreitol) and sodium dithionite; Promega Corp., USA) is added and luminescence is measured after a minimum of 15 min. incubation at RT.

### Materials:

HeLa-R5-16 cells (cell line to express HIV gp160 upon doxycycline induction) are cultured in DMEM medium containing nutrients and 10 % FCS with 400 µg/ml G418 and 200 µg/ml Hygromycin B. CEM.NKR-CD4-Luc (Catalog Number: 5198, a T-cell line available from NIH AIDS Research & Reference Reagent Program McKesson BioServices Corporation Germantown, MD 20874, USA). Cell Type: CEM.NKR-CD4 (Cat. #4376) is transfected (electroporation) to express the luciferase gene under the transcriptional control of the HIV-2 LTR and propagated in RPMI 1640 containing 10 % fetal bovine serum, 4 mM glutamine, penicillin/streptomycin (100 U/mL Penicillin, 100 µg/mL Streptomycin), and 0.8 mg/ml geniticin sulfate (G418). Growth Characteristics: Round lymphoid cells, morphology not very variable. Cells grow in suspension as single cells, which can form small clumps. Split 1:10 twice weekly. Special Characteristics: Express luciferase activity after transactivation of the HIV-2 LTR. Suitable for infection with primary HIV isolates, for neutralization and drug-sensitivity assays (Spenlehauer, C., et al., Virology 280 (2001) 292-300; Trkola, A., et al., J. Virol. 73 (1999) 8966-8974). The cell line was obtained through the NIH AIDS Research and Reference Reagent Program, NIAID, NIH from Drs. John Moore and Catherine Spenlehauer. Bright-Glo™ Luciferase assay buffer (Promega Corp. USA, Part No E2264B), Bright-Glo™, Luciferase assay substrate (Promega Corp. USA, Part No EE26B).

### Example 9

### Antiviral activity assay in peripheral blood mononuclear cells (PBMC).

Human PBMC are isolated from buffy-coats (obtained from the Stanford Blood Center) by a Ficoll-Paque (Amersham, Piscataway, New Jersey, USA) density gradient centrifugation according to manufacturer's protocol. Briefly, blood is transferred from the buffy coats in 50 ml conical tubes and diluted with sterile Dulbecco's phosphate buffered saline (Invitrogen/Gibco) to a final volume of 50 ml. Twenty-five ml of the diluted blood are transferred to two 50 ml conical tubes, carefully underlayerd with 12.5 ml of Ficoll-Paque Plus (Amersham Biosciences) and centrifuged at room temperature for 20 min. at 450 x g without braking. The white cell layer is carefully transferred to a new 50 ml conical tube and washed twice with PBS. To remove remaining red blood cells, cells are incubated for 5 min. at room temperature with ACK lysis buffer (Biosource) and washed one more time with PBS. PBMC are counted and incubated at a concentration of 2-4 x 10⁶ cells/ml in RPMI1640 containing 10 % FCS (Invitrogen/Gibco), 1 % penicillin/streptomycin, 2 mM L-glutamine, 1 mM sodium-pyruvate, and 2 µg/ml Phytohemagglutinin (Invitrogen) for 24 h at 37 °C. Cells are incubated with 5 Units/ml human IL-2 (Roche Molecular Biochemicals) for a minimum of 48 h prior to the assay. In a 96 well round bottom plate, 1 x 10⁵ PBMC are infected with the HIV-1 JR-CSF virus (Koyanagi, Y., et al., Science 236 (1987) 819-822) in the presence of serially diluted test antifusogenic peptide-anti-CD4 antibody-conjugates, reference immunoglobulins, parent ant-CD4 antibody, and reference peptides (T-651). The amount of virus used is equivalent to 1.2 ng HIV-1 p24 antigen/well. Infections are set up in quadruplicates. Plates are incubated for 6 days at 37 °C. Virus production is measured at the end of infection by using p24 ELISA (HIV-1 p24 ELISA #NEK050B, Perkin Elmer/NEN) using the sigmoid dose-response model with one binding site in Microsoft Excel Fit (version 3.0.5 Build 12; equation 205; Microsoft).

**Table 5: Antiviral activity of antifusogenic polypeptides, antibodies and antifusogenic peptide-anti-CD4 antibody conjugates (by weight).**

| **Compound** | **Antiviral activity** | |
|---|---|---|
| | **NL-BAL** | **NL-4-3** |
| | **IC₅₀ [ng/mL] / IC₉₀ [ng/mL]** | **IC₅₀ [ng/mL] / IC₉₀ [ng/mL]** |
| Reference antibody 1 (inert) | inactive / inactive | inactive / inactive |
| Reference antibody 2 (inert) | inactive / inactive | inactive / inactive |
| T-651 | 14.1 / 57.8 | 41.4 / 79.6 |
| afp-1 | - / 57.6 | |
| anti-CD4 antibody (6310/6309) | 917.6 / max. 54 % inhibition | 35.6 / 1117.0 |
| antifusogenic peptide-anti CD4 | 5.3 / 18.2 | 3.2 / 25.1 |
| antibody conjugate (6310/6303) | | |

In Table 6 the antiviral activity of antifusogenic peptide-anti-CD4 antibody conjugates depending on the number of glycosylation sites in the molecule is given. As can be seen with the number of glycosylation sites the antiviral activity varies.

**Table 6: Antiviral activity of antifusogenic polypeptides, antibodies and antifusogenic peptide-anti-CD4 antibody conjugates (by weight).**

| **Compound** | **Antiviral activity** | |
|---|---|---|
| | **NL-BAL** | **NL-4-3** |
| | **IC₅₀ [ng/mL] / IC₉₀ [ng/mL]** | **IC₅₀ [ng/mL] / IC₉₀ [ng/mL]** |
| T-651 | 7.3 / 36.0 | 26.7 / 95.7 |
| anti-CD4 antibody (6310/6309) | 514.4 / > 5000 | 32.7/743.6 |
| one glycosylation site in the heavy chain | | |
| antifusogenic peptide-anti CD4 | 7.1 / 44.0 | 4.2 / 17.54 |
| antibody conjugate (6310/6304) | | |
| one glycosylation site in the heavy chain, two in the antifusogenic peptide | | |
| antifusogenic peptide-anti CD4 | 3.4 / 12.1 | 2.5 / 19.5 |
| antibody conjugate (6310/6303) | | |
| one glycosylation site in the heavy chain, one in the antifusogenic peptide | | |
| antifusogenic peptide-anti CD4 | 1.4 / 8.5 | 1.3 / 7.5 |
| antibody conjugate (6310/6305) | | |
| one glycosylation site in the heavy chain, none in the antifusogenic peptide | | |

## Claims

1. A conjugate comprising two or more antifusogenic peptides and an anti-CD4 antibody, **characterized in that** two to eight antifusogenic peptides are each conjugated to one terminus of the heavy and/or light chains of said anti-CD4 antibody.

2. A conjugate according to claim 1, **characterized in that** the conjugate has the general formula
mAb CD4 - [linker]ₘ - [antifusogenic peptide]ₙ
wherein m is independently for each antifusogenic peptide either 0 or 1,
wherein n is an integer of from 2 to 8.

3. A conjugate according to claim 2, **characterized in that** n is 2 or 4.

4. A conjugate according to claim 1, **characterized in that** the conjugate comprises a heavy and/or light chain of mAb CD4 and an antifusogenic peptide ("chain conjugate") selected from the group consisting of (in N-terminal to C-terminal direction):
(1) [antifusogenic peptide] - [linker]ₘ - [heavy chain],
(2) [heavy chain] - [linker]ₘ - [antifusogenic peptide],
(3) [antifusogenic peptide] - [linker]ₘ - [heavy chain] - [antifusogenic peptide],
(4) [antifusogenic peptide] - [linker]ₘ - [light chain],
(5) [light chain] - [linker]ₘ - [antifusogenic peptide],
(6) [antifusogenic peptide] - [linker]ₘ - [light chain] - [antifusogenic peptide],
(7) [antifusogenic peptide] - [linker]ₘ - [heavy chain] - [linker]ₘ - [antifusogenic peptide],
(8) [antifusogenic peptide] - [linker]ₘ - [light chain] - [linker]ₘ - [antifusogenic peptide],
wherein the linker can be the same or different in said chain conjugates, wherein m is an integer of 1 or 0, and m can be independently the same or different in said chain conjugates.

5. A conjugate according to claim 4 comprising
2x [mAb CD4 light chain] and 2x chain conjugate (2),
2 x [mAb CD4 light chain] and 2 x chain conjugate (3),
2 x [mAb CD4 heavy chain] and 2 x chain conjugate (4), or
2 x [mAb CD4 light chain] and 2 x chain conjugate (7).

6. A conjugate according to claim 1 or 3, **characterized in that** the conjugate comprises a variable heavy chain domain consisting of an immunoglobulin framework and a CDR3 region selected from the CDR3 sequences of SEQ ID NO: 29, 30, or 31, a CDR2 region selected from the CDR2 sequences of SEQ ID NO: 26, 27, or 28, and a CDR1 region selected from the CDR1 sequences of SEQ ID NO: 23, 24, or 25.

7. A conjugate according to claim 1 or 3, **characterized in that** is comprises a variable light chain domain consisting of an immunoglobulin framework and a CDR1 region selected from SEQ ID NO: 13, 14, 15, or 16, a CDR2 region selected from SEQ ID NO: 17, 18, or 19, and a CDR3 region selected from SEQ ID NO: 20, 21, or 22.

8. A conjugate according to claim 1 or 3, **characterized in that** it comprises the heavy chain variable domain defined by amino acid sequence SEQ ID NO: 07, and the light chain variable domain defined by amino acid sequence SEQ ID NO: 01; or the heavy chain variable domain defined by amino acid sequence SEQ ID NO: 08, and the light chain variable domain defined by amino acid sequence SEQ ID NO: 02; or the heavy chain variable domain defined by amino acid sequence SEQ ID NO: 10, and the light chain variable domain defined by amino acid sequence SEQ ID NO: 04; or the heavy chain variable domain defined by amino acid sequence SEQ ID NO: 11, and the light chain variable domain defined by amino acid sequence SEQ ID NO: 05, or the heavy chain variable domain defined by amino acid sequence SEQ ID NO: 12, and the light chain variable domain defined by amino acid sequence SEQ ID NO: 06; a linker selected from the amino acids glycine (G) and asparagine (N), the tripeptide GST, and the SEQ ID NO: 46 to 76 or 80 or 81; and an antifusogenic peptide selected from the antifusogenic peptides of SEQ ID NO: 35 to 44 or 83.

9. A conjugate according to 1 or 3, **characterized in that** the conjugate comprises an antifusogenic peptide selected from the antifusogenic peptides C34, T20, T1249, T651, T2635, N36, DP107, or afp-1, or afp-2.

10. A conjugate according to claim 1 or 3, **characterized in that** it comprises an antifusogenic peptide at each C-terminus of the heavy chains or at each N-terminus of the light chains.

11. A conjugate according to claim 1 or 3, **characterized in that** is comprises two light chain variable domains of SEQ ID NO: 01, two conjugates of type (2) each comprising a heavy chain variable domain of SEQ ID NO: 07, a linker of SEQ ID NO: 75, and an antifusogenic peptide of SEQ ID NO: 42, in comprising two light chain variable domains of SEQ ID NO: 02, two conjugates of type (2) each comprising a heavy chain variable domain of SEQ ID NO: 08, a linker of SEQ ID NO: 75, and an antifusogenic peptide of SEQ ID NO: 42, in comprising two light chain variable domains of SEQ ID NO: 04, two conjugates of type (2) each comprising a heavy chain variable domain of SEQ ID NO: 10, a linker of SEQ ID NO: 75, and an antifusogenic peptide of SEQ ID NO: 42, in comprising two light chain variable domains of SEQ ID NO: 05, two conjugates of type (2) each comprising a heavy chain variable domain of SEQ ID NO: 11, a linker of SEQ ID NO: 75, and an antifusogenic peptide of SEQ ID NO: 42, or in comprising two light chain variable domains of SEQ ID NO: 06, two conjugates of type (2) each comprising a heavy chain variable domain of SEQ ID NO: 12, a linker of SEQ ID NO: 75, and an antifusogenic peptide of SEQ ID NO: 42.

12. A conjugate according to claim 1 or 3, **characterized in that** said anti-CD4 antibody of IgG4 subclass has a S228P mutation and said anti-CD4 antibody of IgG1 subclass has L234A and L235A mutations.

13. A conjugate according to claim 1 or 3, **characterized in that** said anti-CD4 antibody is a human antibody, a humanized antibody, a chimeric antibody, or a T cell antigen depleted antibody.

14. Pharmaceutical composition, containing a conjugate according to claim 1 together with a pharmaceutically acceptable excipient or carrier.

15. Use of a conjugate according to claim 1 for the manufacture of a medicament for the treatment of viral infections.
